**Europäisches Patentamt**

**European Patent Office**

**Office européen des brevets**

(11) Numéro de publication : **0 294 272 B1**

(12) # FASCICULE DE BREVET EUROPEEN

(45) Date de publication du fascicule du brevet :
**18.03.92 Bulletin 92/12**

(51) Int. Cl.⁵ : **A61M 5/30**

(21) Numéro de dépôt : **88401260.0**

(22) Date de dépôt : **24.05.88**

(54) **Appareil d'injection de liquide, sans aiguille notamment à usage de soins dentaires.**

Jointe à la demande no. 88904567.0/0315661
(numéro de dépôt/numéro de publication de la
demande européenne) par décision du
12.09.89.

(30) Priorité : **26.05.87 FR 8707409**
**11.04.88 FR 8804764**

(43) Date de publication de la demande :
**07.12.88 Bulletin 88/49**

(45) Mention de la délivrance du brevet :
**18.03.92 Bulletin 92/12**

(84) Etats contractants désignés :
**AT BE CH DE ES FR GB GR IT LI LU NL SE**

(56) Documents cités :
**FR-A- 2 474 319**
**US-A- 3 130 723**
**US-A- 3 403 680**
**US-A- 3 461 867**
**US-A- 4 165 739**

(73) Titulaire : **Accaries, Claude**
**Route du Pont-Neuf**
**F-64260 Arudy (FR)**
Titulaire : **Ibis, Pierre**
**27 chemin Cam-Loung**
**F-64230 Lescar (FR)**
Titulaire : **Toprides, Henri**
**7 rue de Crillon**
**F-92210 Saint-Cloud (FR)**

(72) Inventeur : **Accaries, Claude**
**Route du Pont-Neuf**
**F-64260 Arudy (FR)**
Inventeur : **Ibis, Pierre**
**27 chemin Cam-Loung**
**F-64230 Lescar (FR)**
Inventeur : **Toprides, Henri**
**7 rue de Crillon**
**F-92210 Saint-Cloud (FR)**

(74) Mandataire : **Armengaud Ainé, Alain et al**
**Cabinet ARMENGAUD AINE 3 Avenue**
**Bugeaud**
**F-75116 Paris (FR)**

## Description

La présente invention est relative à un appareil destiné à l'injection de produits liquides à l'intérieur de la muqueuse dentaire d'un patient au moyen d'un jet sous pression réglable pénétrant directement dans la gencive.

On sait que dans de nombreuses interventions pratiquées par les chirurgiens dentistes sur la dentition d'un individu, notamment pour réaliser une anesthésie locale avant l'extraction d'une dent ou plus généralement pour l'administration à l'intérieur de la muqueuse dentaire de substances médicamenteuses appropriées à un traitement déterminé, il est nécessaire de pratiquer une injection d'un produit liquide en quantité exactement contrôlée, avec une fréquence qui, le cas échéant, peut être relativement élevée au cours de l'intervention. A cet effet, on connaît déjà des appareils dont la forme, adaptée à la pratique dentaire et qui présentent une ergonomie adéquate pour faciliter leur manipulation par le praticien, n'en sont pas moins directement dérivés des seringues classiques où le liquide, poussé par un piston se déplaçant dans une cavité, est chassé à travers le canal interne d'une aiguille creuse, qui est préalablement introduite par son extrémité ouverte et tranchante dans la muqueuse, à l'endroit où le produit doit être injecté. Ces appareils classiques sont cependant peu pratiques et nécessitent notamment une consommation d'aiguilles dont le renouvellement pour chaque intervention, voire pour chaque injection, est indispensable. De plus, l'utilisation d'une aiguille fixée à l'extrémité d'une seringue a toujours pour le patient un aspect traumatisant, que certains ne supportent qu'avec de grandes difficultés. Par ailleurs, l'utilisation d'un appareil de ce genre exige de la part d'un praticien une grande dextérité, surtout si l'endroit de la gencive où doit être injecté le produit n'est pas d'un accès facile, par exemple à l'intérieur et au fond de la cavité buccale ou au voisinage de la face linguale des incisives, l'encombrement de l'aiguille rendant cette opération d'autant plus délicate que la région choisie est ainsi plus difficile à atteindre.

Enfin, il n'est pas nécessaire d'insister sur les risques qui résultent d'une contamination possible du patient, voire du praticien lui-même, par le sang qui accompagne souvent la pénétration de l'aiguille dans la muqueuse, ce qui constitue à nouveau un inconvénient encore plus important pour la mise en oeuvre des appareils d'injection classiques, utilisant de telles aiguilles.

Pour pallier cet inconvénient, on a déjà prévu de substituer à la seringue mentionnée ci-dessus, un dispositif injecteur sans aiguille, du genre général de ceux utilisés dans la technique de soin connue sous le nom de mésothérapie. Toutefois, avec l'appareil correspondant, l'armement du piston qui créé le jet liquide extrêmement fin pénétrant directement dans la gencive par la seule force de son impact au contact de celle-ci, s'effectue au moyen d'un levier manuel qui doit être manoeuvré à chaque injection. Il en résulte un appareil lourd, peu commode à manipuler et qui, surtout, ne permet pas de faire varier la dose à injecter qui est toujours la même, la course du piston étant en permanence identique à elle-même. De plus, pour l'injection de doses notables, afin par exemple de réaliser une anesthésie locale profonde, la pénétration de la quantité utile du produit conduit à effectuer une série de manoeuvres successives du levier d'armement de l'appareil, ce qui rend celui-ci peu pratiquable dans des conditions d'efficacité satisfaisantes.

Un autre inconvénient enfin, résulte du fait que, dans certaines réalisations, le liquide à injecter doit être, préalablement à l'emploi de l'appareil, transféré dans un réservoir monté à l'intérieur de celui-ci, ce qui pose problème pour nettoyer et le cas échéant aseptiser ce dernier lors d'un changement de produit, avec en outre l'inconvénient d'une consommation notable de celui-ci, le surplus devant normalement être à chaque fois jeté.

On connaît par ailleurs par le FR-A- 2 474 319 un appareil d'injection de liquide sans aiguille comportant un corps allongé en deux parties séparables placées bout à bout. Dans la première partie est montée une carpule contenant le liquide à injecter et communiquant avec une chambre de dosage à volume variable, elle-même reliée à une buse munie d'un injecteur propre à former un jet de liquide étroit et sous pression, consécutivement à un brutal refoulement du liquide hors de cette chambre. La seconde partie comprend un ensemble mobile à mouvement alternatif, comportant un piston de travail et un ressort de commande et de propulsion agissant sur ce piston en état progressivement comprimé sous l'effet du déplacement d'une pièce de poussée provoquée par une arrivée d'un liquide hydraulique, notamment de l'huile sous pression, à l'intérieur d'une chambre étanche prévue dans le corps de l'appareil. Celui-ci comporte une gâchette qui permet d'actionner un clapet qui libère la pression dans la chambre étanche et autorise la détente du ressort de commande et par suite le déplacement du piston de travail.

Or, un tel système présente l'inconvénient que la formation du jet de liquide résulte d'une action manuelle sur la gâchette, ce qui ne permet pas de déterminer avec une grande précision le moment exact où est réalisée l'injection du liquide, surtout si l'appareil doit permettre un fonctionnement rapide avec plusieurs injections successives effectuées dans un temps très bref, ce qui exige que le réarmement du piston de travail soit lui-même obtenu dans un laps de temps extrêmement réduit. L'appareil selon ce brevet antérieur n'est donc pas adapté pour une application à des soins dentaires que vise plus particulièrement la présente invention. En outre, l'usage d'un fluide liquide, et plus spécialement de l'huile, pour la commande du piston, est totalement exclu dans une telle application.

La présente invention est relative à un appareil d'injection sans aiguille, notamment pour application à l'art dentaire, qui pallie les inconvénients des solutions de la technique antérieure, en particulier en permettant la création d'un jet sous pression réglable, répétitif avec une cadence pouvant être ajustée à volonté, le fonctionnement de l'appareil étant entièrement pneumatique et agencé de telle sorte qu'il réalise un retour automatique de l'ensemble mobile assurant la formation du jet liquide à sa position initiale quand cet ensemble a atteint une position déterminée pendant son mouvement d'armement, la pression de gaz, en particulier d'air, nécessaire au fonctionnement étant commandé par une simple pression fournie par l'opérateur lui-même, agissant sur un organe de manoeuvre, par exemple une pédale ou tout autre moyen analogue, auquel est raccordé le pupitre de commande fournissant l'air comprimé disponible de manière habituelle dans mes installations de chirurgie dentaire.

L'appareil selon l'invention est par ailleurs conçu de telle sorte que sa forme puisse être bien adaptée à son usage, en autorisant sa tenue par la main du dentiste de manière tout à fait similaire à celle qu'il a coutume de rencontrer avec ses autres instruments habituels, du type fraise rotative, pulvérisateur d'eau ou d'air, etc, disponibles sur son pupitre de trzavail, ceci grâce à une conception des organes mécvaniques de l'appareil et de leur cinématique de fonctionnement, permettant de les loger dans un ensemble cylindrique de faible poids, présentant un diamètre limité et un encombrement total très réduit.

A cet effet, l'appareil d'injection considéré, comportant un corps allongé en deux parties séparables placées bout à bout, dont la première dite médicale, comprend au moins un logement recevant une carpule étanche contenant le liquide à injecter et au moins une chambre de dosage à volume variable, ménagée dans ladite première partie et communiquant avec une buse munie d'un injecteur propre à former un jet de liquide étroit consécutivement à un brutal refoulement du liquide hors de la chambre de dosage et dont la seconde partie, dite moteur, comporte un ensemble mobile à mouvement alternatif comprenant un piston de travail et un ressort de commande et de propulsion, agissant sur ce piston en étant progressivement comprimé sous l'effet du déplacement d'une pièce de poussée, provoqué par une pression d'un fluide de propulsion dans une chambre étanche prévue dans le corps, caractérisé en ce que la carpule présente un fond mobile dont le déplacement réalise l'envoi de liquide depuis cette carpule vers la chambre de dosage, en ce que le fluide de propulsion est de l'air comprimé et en ce que le déplacement continu de la pièce de poussée se produit jusqu'au moment où le ressort est automatiquement et brutalement libéré au moyen d'un organe mobile, à déplacement limité, autorisant le retour rapide du piston simultanément avec l'extrusion du liquide hors de la chambre de dosage, préalablement au retour en position initiale de la pièce de poussée.

L'invention vise plusieurs modes d'exécution de l'organe mobile à déplacement limité provoqué par la pièce de poussée agissant sur le piston de travail afin de permettre d'assurer automatiquement et brutalement la libération de ce dernier et l'expulsion du liquide préalablement dosé.

Dans une première variante de réalisation de l'appareil, considéré, l'ensemble mobile comprend un piston de travail faisant également office de pièce de poussée, le piston séparant l'intérieur du corps dans la seconde partie en deux chambres disposées de part et d'autre du piston de travail et dont la première est délimitée partiellement par le fond mobile de la carpule et est progressivement mise sous pression d'air comprimé de manière à déplacer le piston de travail en sens inverse à l'encontre du ressort de commande et de propulsion logé dans la seconde chambre, ledit ressort provoquant le retour rapide du piston de travail suite à une soudaine réduction de la pression dans ladite première chambre commandée par un distributeur coaxial au piston de travail et déplacé sur une très faible distance par le piston en fin de course de façon à mettre la pression dans la première chambre à l'échappement à l'air libre, le piston de travail dans son retour rapide transmettant son mouvement à un piston d'expulsion mobile dans la chambre de dosage et qui chasse le liquide hors de celle-ci.

Selon une autre caractéristique de l'appareil considéré, le piston d'expulsion du liquide hors de la chambre de dosage comporte une tête munie d'un organe d'étanchéité et montée coulissante dans ladite chambre, ladite tête étant prolongée par une tige, éventuellement en plusiers éléments successifs pour ajuster la longueur de ladite tige, le piston d'expulsion étant soumis à l'effet permanent d'un ressort auxiliaire qui repousse la tige contre un embout coaxial, solidaire du piston de travail et qui est en contact avec l'extrémité de la tige lorsque les deux parties du corps sont assemblées.

Dans une autre variante, qui permet l'avantage d'une structure plus simple et en particulier plus facile à réaliser industriellement grâce à une configuration de ses diverses parties, aussi bien fixes que mobiles, qui soit telle qu'elle présente pour l'essentiel un profil de révolution autour de l'axe du corps, le piston de travail de l'ensemble mobile est soumis à l'action du ressort de commande progressivement comprimé par une pièce de poussée axiale séparée réunie par une membrane élastique à la paroi interne d'une chambre de compression reliée à une source d'air comprimé, des moyens étant prévus pour solidariser le piston de travail avec un organe d'immobilisation temporaire prévu dans le corps, coaxialement au piston, de telle sorte que, au-delà d'une course déterminée de la pièce de poussée, provoquant la compression progressive du ressort, celui-ci soit brutalement libéré et détermine le déplacement rapide du piston, en réalisant l'expulsion hors de la chambre de dosage du liquide à injecter, communiquant avec la carpule formant réserve de liquide.

Selon le cas, le piston mobile fait ou non partie d'un ensemble qui inclut la carpule elle-même, celle-ci étant donc immobilisée dans le corps ou au contraire déplaçable dans celui-ci avec le piston, la brutale expulsion du liquide hors de la chambre de dosage vers la buse d'injection pouvant s'effectuer consécutivement à une action directe ou indirecte de ce piston.

Avantageusement, les moyens de blocage sont constitués par des billes montées dans des logements ouverts prévus dans la paroi externe du piston et maintenus en position par un manchon coaxial, coulissant sur cette paroi sous l'effet de la pièce de poussée, ce manchon comportant un évidement profilé pour l'échappement des billes et la libération du piston au-delà d'une course donnée du manchon.

D'autres caractéristiques d'un appareil d'injection établi conformément à l'invention, permettant de faire apparaître plus complètement les avantages qu'il procure vis-à-vis des solutions antérieures déjà connues dans la technique, apparaîtront encore à travers la description qui suit, de plusieurs exemples de réalisation, donnés à titre indicatif et non limitatif, en référence aux dessins annexés sur lesquels :

La figure 1 est une vue extérieure schématique d'ensemble d'une première variante de l'appareil considéré, avec ses deux parties assemblées l'une à l'autre.

Les figures 2a et 2b sont des vues en coupe longitudinales à plus grande échelle de l'appareil selon la figure 1, illustrant plus particulièrement le détail de la réalisation mécanique des deux parties du corps de l'appareil, et permettant d'expliciter le fonctionnement de celui-ci.

La figure 3 est une vue en coupe de la figure 2b selon la ligne III-III de cette dernière.

La figure 4 est une vue de détail de la figure 2b, montrant le déplacement du distributeur.

La figure 5 est une vue de détail d'une variante de réalisation de l'extrémité de l'appareil, portant la buse d'injection.

Les figures 6a et 6b sont des vues de deux parties consécutives de l'appareil selon l'invention dans une autre variante de réalisation de celui-ci.

La figure 7 est une vue en coupe transversale à légèrement plus grande échelle, selon la ligne II-II de la figure 6b.

Les figures 8 et 9 sont des vues en coupe longitudinale partielle à plus grande échelle des organes permettant d'assurer le blocage puis le déblocage du piston de travail de l'appareil consécutivement à l'effort exercé sur celui-ci par la pièce de poussée, elle-même actionnée par une pression commandée d'air comprimé.

La figure 10 est une autre vue en coupe partielle d'une variante d'exécution des moyens permettant d'exercer sur le liquide contenu dans la carpule de l'appareil, la pression nécessaire à l'écoulement de ce liquide vers la chambre de dosage.

Les figures 11 et 12 sont des vues respectivement en coupes transversale et longitudinale, d'une variante de réalisation d'un dispositif permettant d'immobiliser le piston de travail jusqu'à ce que l'appareil ait été mis convenablement en position, avant la réalisation de l'injection d'un jet liquide.

La figure 13 illustre une variante de réalisation du dispositif des figures 11 et 12.

Les figures 14 et 15 d'une part, 16 puis 17 d'autre part, illustrent respectivement et de façon schématique trois autres variantes de réalisation du dispositif de blocage associé à l'appareil selon l'invention.

Les figures 18a et 18b représentent les deux parties consécutives de l'appareil selon une autre variante générale de celui-ci.

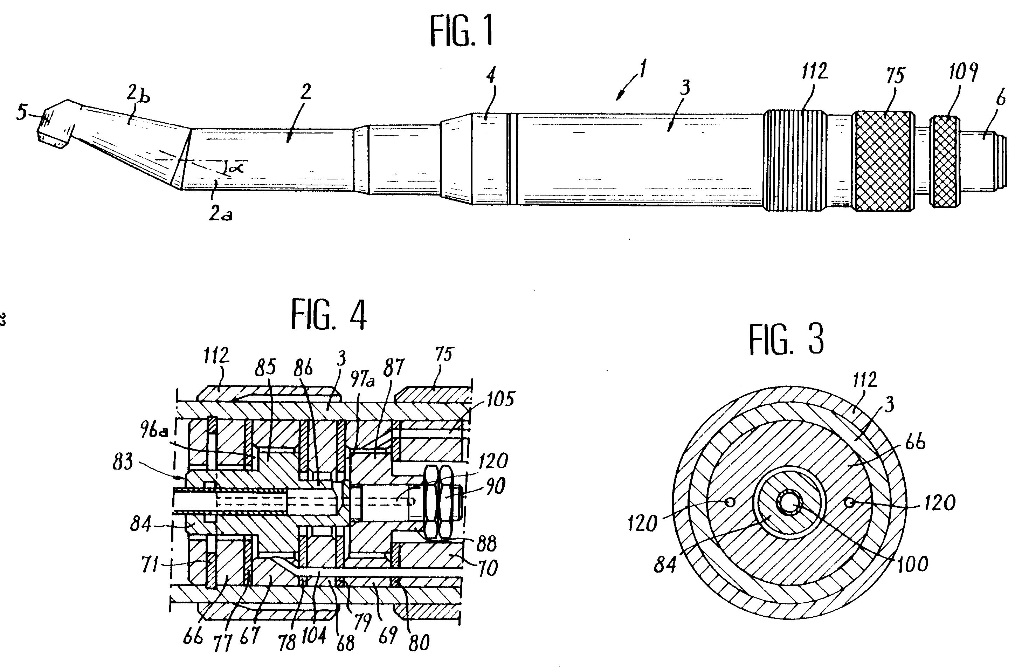

L'appareil selon l'invention tel qu'illustré sur la figure 1 en vue extérieure, comporte essentiellement un corps allongé 1 en deux parties 2 et 3 séparables, réunies l'une à l'autre en fonctionnement par une bague de liaison filetée 4. La partie 2 du corps, dite partie médicale, est formée de deux éléments respectivement 2a et 2b dont le premier est situé dans l'axe de la seconde partie 3, dite partie moteur, tandis que l'élément 2b présente une direction axiale faisant avec celle de l'élément 2a un angle de 10 à 15 degrés, facilitant l'emploi de l'appareil pour le praticien qui peut introduire celui-ci plus ou moins profondément dans la bouche du patient selon l'endroit où il souhaite pratiquer une injection de liquide. La partie 2b comporte à son extrémité opposée à la partie 2a, une tête 5, munie d'une buse d'injection, propre à former de la manière qui sera indiquée plus loin, un jet de liquide très mince et de grande puissance, dont la conformation lui permet de pénétrer directement la gencive du patient sans l'aide d'aucune aiguille ou élément analogue. A l'opposé de la tête 5, la partie moteur 3 du corps 1 comporte un raccord de liaison 6 avec une prise d'air comprimé, d'un usage classique dans les pupitres des installations de chirurgie dentaire. La pression de l'air comprimé fourni est généralement réglée entre 2 et 7 bars.

Les figures 2a, 2b et 3 illustrent avec plus de détails la structure des deux parties 2 et 3 du corps 1 de l'appareil.

Comme on le voit sur la figure 2a, la partie 2 présente aussi un logement interne 7, à l'intérieur duquel peut être engagée une carpule ou conteneur d'un type classique 8, présentant notamment une enveloppe 9 dans laquelle est enfermée une quantité d'un liquide dont on souhaite au moyen de l'appareil considéré provoquer l'injection à dose exactement déterminée dans la muqueuse gingivale d'un patient. L'enveloppe 9 de la carpule 8 présente à son extrémité dirigée vers le fond du logement 7 quand cette carpule est introduite dans celui-ci, un rétreint délimitant un goulot 10 sur le bord duquel s'applique une plaquette souple 11, en caoutchouc ou autre matériau du même genre, fermant de façon étanche l'enveloppe 9. La plaquette souple 11 est immobilisée sur le goulot 10 de façon connue par une capsule de sertissage 12, percée en son centre. A son extrémité opposée, l'enveloppe 9 comporte une pièce de fond cylindrique 13, munie dans sa surface latérale de segments d'étanchéité 14, cette pièce de fonds 13 étant susceptible de se mouvoir dans l'enveloppe 9 pour provoquer progressivement, au fur et à mesure de son déplacement, la sortie hors de la carpule d'une quantité donnée du liquide 15 qu'elle contient, à travers un passage de sortie préalablement ménagé dans la plaquette étanche 11. Une fenêtre 7a est avantageusement prévue dans la paroi de la partie 2 au droit du logement 7 permettant de contrôler la quantité de liquide restant dans la carpule.

La carpule 8 ainsi montée dans le logement 7 est en appui contre le fond de ce dernier sur la pièce 17, vissée au moyen d'une fente transversale 16 prévue sur l'extrémité de cette pièce dans un trou taraudé 18 prévu dans la partie 2a de l'élément 2, en regard du logement. La pièce 17 est percée d'un conduit 19. A l'extrémité de celui-ci dirigée vers l'intérieur du logement 7, est engagé le bout fileté 20 ou emmanché à force d'un trocart 21 qui vient ainsi percer la plaquette d'étanchéité 11 de la carpule par son extrémité biseautée coupante 22, lorsque cette carpule est introduite dans le logement 7. A son extrémité opposée, le conduit 19 débouche dans une cavité élargie 23, délimitée par une bague d'appui creuse 25 et dans laquelle est montée libre une bille 24, formant clapet de fermeture de ce conduit lorsque cette bille est immobilisée contre la sortie de celui-ci dans les conditions qui seront exposées plus loin. La cavité 23 présente de préférence une section carrée, triangulaire ou autre de telle sorte que lorsque la bille 24 s'appuie contre son extrémité sur la bague 25, le passage à travers celle-ci ne soit pas complètement obturé, en permettant au liquide soutiré de la carpule selon le processus décrit ultérieurement, de s'écouler à travers la cavité 23 et la bague 25. Cette cavité 23 est par ailleurs raccordée, avec un canal de liaison 26 débouchant dans un évidement 27 ménagé dans la partie 2b de l'élément 2 au droit de la zone où celui-ci se raccorde à la partie 2a. Cet évidement 27 communique par ailleurs, dans les parties 2a et 2b respectivement avec deux conduits de liaison 28 et 29, disposés dans le prolongement l'un de l'autre et permettant d'acheminer à la tête d'injection 5 une quantité déterminée de liquide soutirée de la carpule 8 et renvoyée vers la tête avec la pression nécessaire à cette injection.

Dans la tête 5, le conduit 29 est réuni à un élément coudé à angle droit 30, en bout duquel est monté un second clapet, constitué à nouveau par une bille 31, appliquée sur l'extrémité du coude 30 par un ressort 32. Celui-ci s'appuie à l'opposé sur l'arrière d'une buse amovible 33, vissée dans le filetage interne d'un logement borgne 34, ces dispositions permettant un changement facile de la buse en cas d'usure ou de détérioration. La buse 33 présente un évidement axial 35 et, en fond de celui-ci, un injecteur 36, constitué par une micro-plaquette de rubis ou autre matériau très dur et résistant en particulier à l'effet d'abrasion provoqué par le jet de liquide sous pression, cette microplaquette étant percée axialement d'un trou calibré de très faible diamètre, de l'ordre de quelques centièmes à quelques dixièmes de millimètres selon la taille du jet à réaliser et la nature du liquide à injecter.

De préférence, un capuchon de protection 37 en matière plastique ou autre, entoure l'extrémité de l'élément 2b de la partie 2, ce capuchon présentant, au droit de la buse 33, une ouverture appropriée afin de ne pas gêner la sortie du jet fourni par la buse. Le capuchon 37 est interchangeable et peut, en particulier, être aisément jeté après chaque utilisation de l'appareil ou intervention sur un patient.

Le conduit 28, opposé au conduit 29 décrit ci-dessus, débouche pour sa part à l'intérieur de l'élément 2a dans une chambre 38 de dosage du liquide à injecter. Dans cette chambre 38, est monté un joint à lèvre 39 fixé en bout de la tête d'un piston 40 dit piston d'expulsion ou de refoulement, agencé de façon à permettre, selon le sens de son déplacement dans la chambre 38, le remplissage ou l'éjection d'un volume de liquide, respectivement aspiré ou expulsé de la chambre. La tête de piston 40 comporte par ailleurs dans sa partie médiane un épaulement 41, monté coulissant dans un chambrage 42 prévu à l'intérieur de la partie 2 du corps 1 à côté du logement 7 et dans lequel est logé un ressort 43 en appui d'une part contre le fond de ce chambrage 42 et d'autre part sous l'épaulement 41, de telle sorte que la tête de piston 40 soit en permanence repoussée en direction de la partie 3 du corps 1. La tête de piston 40 se prolonge au delà de l'épaulement 41, par une tige axiale 44, éventuellement réalisée en plusieurs éléments 44a, 44b, etc, de longueur variable de façon à ajuster la dimension totale de la tige 44, ceci afin de permettre, comme on le verra plus loin, de régler le volume d'aspiration du liquide dans la chambre de dosage 38 et, selon la longueur de la tige, de modifier ce volume en fonction de la dose à injecter à chaque cycle de fonctionnement du piston et de la nature du liquide utilisé. La tige 44 dont la longueur est ainsi adaptée, vient en butée par son extrémité opposée à l'épaulement 41 contre un embout terminal 45, coaxial à la tige et monté à coulissement dans un alésage adéquat, prévu dans une pièce de fermeture 46. Un joint 45a assure l'étanchéité de l'embout 45 à la traversée de la pièce 46. Celle-ci comporte un double filetage externe 47 permettant d'une part de la fixer en haut de la partie 3 du corps 1 qu'elle ferme ainsi de façon étanche et d'autre part de visser sur elle la bague 4 reliant les deux parties 2 et 3 (figure 1). La bague 4 est arrêtée en translation sur la partie 2 grâce à un rebord en saillie 48 prévu sur la surface externe de celle-ci, en assurant par le vissage de la bague l'accouplement des deux parties jusqu'à butée finale de l'une sur l'autre.

La pièce de fermeture 46 coiffe l'extrémité de la carpule 8 quand les deux parties 2 et 3 du corps 1 sont ainsi réunies dans le prolongement l'une de l'autre par la bague 4. A l'intérieur de cette pièce est montée une rondelle de butée 49, traversée par un passage axial 50 permettant à de l'air comprimé, introduit sous pression dans une première chambre 51 située dans la partie 3 du corps 1 derrière la pièce 46 d'exercer une poussée déterminée sur le fond mobile 13 de la carpule afin de permettre le refoulement hors de celle-ci d'une quantité définie de liquide qui vient se recueillir dans la chambre de dosage 38 de la façon exposée ultérieurement.

La chambre 51 formée derrière la pièce de fermeture 46 est par ailleurs délimitée dans la partie moteur 3 du corps 1 au moyen d'un piston de travail 52 formant pièce de poussée ainsi qu'on le verra plus loin et qui présente notamment dans sa face en regard, une rainure en creux 53, adaptée à recevoir la tête 54 de l'embout 45 de manière à rendre celui-ci solidaire de ce piston de travail 52 en assurant en particulier que le déplacement de cet embout s'effectue en même temps et avec celui de ce piston 52. Celui-ci comporte extérieurement des segments 55 et 56 et, dans une rainure latérale ouverte, un joint souple 57 appliquant un patin 58 contre la surface interne de la partie 3 du corps de manière à assurer en permanence l'étanchéité de la chambre 51 quels que soient les déplacements du piston 52. Celui-ci comporte dans sa face opposée à la rainure 53, donc dans sa face dirigée vers l'arrière du corps 1 un évidement interne 59, dans l'axe duquel est monté un raccord creux 60, prolongé par une extension 61 emmanchée à force à l'intérieur du piston. Le raccord 60 et son extension 61 sont alésés axialement pour permettre d'alimenter la chambre 51 en air comprimé.

Entre le raccord 60 et la paroi interne de l'évidement 59 est monté un fort ressort de commande et de propulsion 62, en appui d'une part contre une portée interne 63 prévue dans le fond de l'évidement 59 à l'intérieur du piston 52 et d'autre part contre une face d'appui 64 d'un bloc support 65, immobilisé à l'intérieur de la partie 3. Ce bloc 65 est formé d'une succession de bagues cylindriques accolées respectivement 66, 67, 68, 69 et 70, engagées successivement à force ou à glissement à l'intérieur du corps 1. La première bague 66 est immobilisée en position par un circlips 71. A l'opposé, la dernière bague 70 comporte un prolongement axial 72 qui fait saillie à l'extérieur de la partie 3 en bout du corps 1, ce prolongement 72 comportant un épaulement 73 venant en butée contre l'extrémité correspondante 74 de cette partie 3 enfermant ainsi cette dernière. Le blocage de l'ensemble des bagues du bloc support 65, une fois celui-ci mis en place dans le corps 1, est réalisée au moyen d'une bague 75 vissée sur un filetage externe 76, prévu sur la surface extérieure de la partie 3 (figures 1 et 2b).

Entre les bagues 66 à 70 du bloc support 65 sont par ailleurs montés des joints plats, désignés sur le dessin sous les références successives 77, 78, 79, 80 et 81, le premier joint 77 étant monté entre les bagues 66 et 67, le second 78 entre les bagues 67 et 68 et ainsi de suite.

Le bloc support avec ses bagues et ses joints plats comporte également un passage axial étagé 82, notamment destiné au montage à l'intérieur du bloc fixe 65 d'un distributeur mobile 83.

Ce dernier est formé pour raison de montage dans le bloc de deux pièces accolées successives dont la première comporte une partie cylindrique 84 prolongée par une extension de plus grand diamètre 85, elle-même terminée par un prolongement cylindrique plus étroit 86. La seconde partie du distributeur 83 comporte de même une partie cylindrique 87, à son tour prolongée vers l'arrière du corps 1 par une extension 88, l'ensemble étant emmanché sur un axe 89 puis bloqué sur celui-ci par un double écrou 90.

Le montage du distributeur 83 à l'intérieur du passage 82 dans le bloc-support 65 délimite ainsi, entre les différentes bagues de ce bloc et les parties correspondantes du distributeur, des jeux annulaires successifs, repérés sur le dessin sous les références 91, 92, 93, 94, la pièce terminale 72 ménageant axialement un logement borgne 95 autour de l'écrou 90. En outre, l'agencement des différents éléments est tel que soit prévu, dans la position du distributeur 83 telle que représentée sur la figure 2b, un jeu axial 96 laissé libre entre l'extension cylindrique 85 et le joint plat 78 d'une part, et un jeu axial de même largeur 97 entre la partie cylindrique 87 et le joint plat 80. Ces jeux 96 et 97 qui communiquent respectivement avec les jeux 92 et 94 définissent notamment la course possible et limitée du distributeur 83 : ainsi, lorsque par suite du déplacement de ce distributeur vers l'arrière du corps 1 à l'intérieur du bloc-support 65 dans le passage 82, les jeux 96 et 97 viennent à disparaître, les parties 85 et 87 venant alors au contact des joints plats 78 et 80, apparaissent simultanément des jeux identiques, respectivement 96a et 97a (figure 4), entre ces mêmes parties cylindriques 85 et 87 et les joints plats 77 et 79.

Un ressort 98 prévu dans le logement 95 de la pièce terminale 72 derrière le distributeur 83 permet en permanence d'exercer sur celui-ci une force de rappel, dirigée de la droite vers la gauche sur le dessin, tendant ainsi à ramener le distributeur dans la position illustrée sur la figure 2b où existent les jeux 96 et 97, les jeux 96a et 97a sont au contraire supprimés.

Le raccord axial 60 du piston 52 comporte un tube de liaison 99 monté coulissant à l'intérieur d'un alésage 100 prévu axialement dans le distributeur 83, avec interposition d'un joint d'étanchéité torique 100a sur lequel peut coulisser ce tube 99.

L'alésage 100 du distributeur 83 communique par un conduit radial 101 avec le jeu annulaire 93 prévu entre le prolongement 86 et la bague fixe 68. A l'extrémité du corps 1, la pièce terminale du bloc-support 65 comporte par ailleurs un évidement ouvert 72a dans lequel débouche une tubulure de raccordement 102 avec une source d'air comprimé à pression comprise entre 2 et 7 bars, disponible de façon classique dans les pupitres des appareils de chirurgie dentaire. Cette tubulure 102 se raccorde dans la pièce 72 à un conduit 103 prolongé par un conduit de liaison 104 traversant successivement les bagues 70, 69 et 68 avant de venir déboucher à travers la bague 67 dans le jeu annulaire 92. De même, à travers les bagues 69, 70 et la pièce terminale 72, est également prévu un second conduit 105 reliant le jeu annulaire 94 à une cavité 106 ouverte vers l'extérieur prévue à l'arrière de la partie 3 du corps 1. Cette cavité 106 a son ouverture ou fente de sortie annulaire 107 partiellement obturée par une partie conique 108 ménagée en bout d'une bague 109 (figures 1 et 2b), propre à se visser sur un filetage 110 ménagé sur la surface externe de la pièce terminale 72. Le vissage progressif de la bague 109 permet à sa partie conique 108 de pénétrer plus ou moins à l'intérieur de la cavité 106 et ainsi de faire varier l'ouverture du jeu 107.

L'équipement de l'appareil se complète en disposant dans la cavité 72a prévue en bout de la pièce terminale 72 un pion de guidage 111, autorisant le raccordement de la tubulure 102 d'arrivée d'air comprimé à une canalisation appropriée. En outre, le corps 1 est avantageusement muni d'un organe de sécurité, constitué par une bague coulissante 112, montée mobile entre deux positions sur la surface externe de la partie 3 dont l'une est définie par une butée sur un épaulement d'arrêt fixe 113. Cette bague 112 comporte intérieurement un passage évidé 114, terminée par une face inclinée 115. Selon la position de la bague 112, cette face inclinée 115 vient ou non agir sur la tête 116 d'une soupape de sécurité 117 soumise à l'action d'un ressort 118 et agencée selon que la bague 112 la maintient enfoncée dans la partie 3 du corps ou au contraire l'autorise à faire saillie hors de celle-ci pour mettre à l'air ou laisser fermé le jeu 92 dans lequel débouche l'extrémité de cette soupape. Enfin, entre la chambre 119 située du côté opposé du piston 52 vis-à-vis de la chambre 51 et le logement 95 est prévu au moins un conduit de communication 120 (en fait deux conduits sont visibles sur la figure 3).

Le fonctionnement de l'appareil d'injection sans aiguille décrit ci-dessus est le suivant : dans un premier temps, on prépare l'appareil en disposant dans le logement, à l'intérieur de la partie 2 ou partie médicale du corps 1 alors séparée de la partie 3, une carpule 8, contenant un liquide 15 que l'on désire faire pénétrer à l'intérieur de la muqueuse dentaire d'un patient en une ou de préférence en plusieurs injections successives. La carpule mise en place, elle est appliquée contre le fond du logement de façon que le trocart 21, par son extrémité coupante et biseautée 22, perfore la rondelle d'étanchéité 11 et rende possible, sous l'effet d'une pression exercée sur le fond mobile 13 de la carpule l'écoulement du liquide 15 à travers le conduit 19 vers la cavité 27 et de là par les conduits 28 et 29 respectivement, soit vers la buse d'injection 33, soit vers la chambre de dosage 38. Une fois la partie 2 ainsi garnie, on assemble celle-ci avec la partie 3, ou partie moteur, par le vissage appropriée de la bague 4. Cette opération permet notamment à l'extrémité de la tige 44 prolongeant la tête du piston de refoulement 40, repoussée par le ressort 43 agissant sur l'épaulement 41, de se maintenir en contact permanent avec l'extrémité correspondante de l'embout 45, solidaire du piston 52 dans la partie 3 ainsi réunie à la partie 2. A noter que l'appareil est agencé pour permettre d'ajuster le réglage initial du volume de la chambre de dosage 38, en jouant sur la longueur de la tige 44 qui peut être modifiée à volonté, en ajoutant ou retirant un ou plusieurs des éléments 44a, 44b, etc., qui mis bout à bout prolongent la tige de façon déjà indiquée.

L'appareil est alors prêt à fonctionner. Il suffit au praticien, tenant en main celui-ci, d'appuyer sur une pédale ou autre moyen de commande analogue pour déclencher la mise en route de la turbine d'admission d'air comprimé à l'appareil, de telle sorte que cet air arrivant par la tubulure 102, soit amené par le conduit 103 et le jeu 92 au distributeur 83 qui, dans la position initiale, est tel que représenté sur la figure 2b. L'air comprimé sous pression passe par le jeu radial 96 puis se sépare à travers le jeu annulaire 93, et de là dans le conduit radial de liaison 101 en étant ainsi amené dans l'alésage axial 100 du distributeur. A travers celui-ci, il passe dans le conduit de liaison 99, puis dans la chambre 51 et enfin à travers le passage 50 vient exercer sa pression contre le fond mobile 13 de la carpule 8 d'une part, en contre la face en regard du piston de travail 52. Simultanément, la chambre 119 située de l'autre côté du piston 52 est mise en communication avec le logement 95 par l'intermédiaire des conduits de liaison 120 (figure 3), et elle est, dans ces conditions, mise à l'air libre, à travers le jeu radial 97, le jeu annulaire 94, le conduit 105, la cavité 106 et la fente réglable de sortie 107.

La pression de l'air comprimé ainsi admise dans la chambre 51 provoque le déplacement du fond mobile 13 de la carpule 8 en expulsant hors de celle-ci la quantité de liquide 15 juste nécessaire pour remplir, après passage dans la bague 25, le conduit 26, l'évidement 27 et le conduit d'air 28, la chambre de dosage 38, ceci au fur et à mesure que le volume de cette dernière s'accroît sous l'effet du déplacement concomitant du piston 52 qui, par l'embout 45 dont il est solidaire, permet à la tige 44 et par suite à la tête du piston de refoulement 40, de se déplacer de la gauche vers la droite sur le dessin de la figure 2b. Au cours de ce déplacement, le liquide 15 aspiré hors de la carpule 8, à travers le trocart 21, la cavité 23 et le conduit 28 vient remplir la chambre de dosage, la bille 24 formant clapet étant repoussée du conduit 19. En revanche, ce liquide ne peut s'échapper par la buse d'injection 33, l'accès à cette dernière étant interdit par la bille 31 du second clapet appliquée contre l'extrémité du coude 30 par le ressort 32.

Tant que se maintient la pression de l'air comprimé dans la chambre 51, le mouvement de déplacement, de la gauche vers la droite, sur le dessin de la figure 2b du piston de travail 52 formant pièce de poussée se poursuit à l'intérieur de la partie moteur 3 du corps 1, l'étanchéité au cours de ce déplacement entre les chambres 51 et 119 respectivement étant maintenue par les segments 55, 56 et le patin 58. Au cours de ce mouvement, le ressort de commande et de propulsion 62 est progressivement bandé dans la chambre 119 qui communique à l'air libre par les conduits 120, les jeux 97, 93, le conduit 105 et la fente 107, jusqu'à ce que le raccord 60 vienne en contact avec l'extrémité de la partie 84 du distributeur 83 qui fait légèrement saillie à l'intérieur de cette chambre. A cet instant, le piston 52 continuant sa course, agit sur le distributeur 83 qu'il repousse légèrement en faisant disparaître les jeux radiaux 96 et 97 et en créant simultanément les jeux 96a et 97a (figure 4). Les extensions 85 et 87 du distributeur quittent leur appui sur les joints 77 et 79 du bloc-support 65, et viennent au contact des joints 78 et 80.

Simultanément, la chambre 51 est mise à l'air libre, l'air comprimé qu'elle contient étant directement mis en communication par l'alésage 100, le conduit radial 101, le jeu annulaire 93, le jeu radial 97a avec le conduit 105, la cavité 106 et la fente 107. Dans ces conditions, le ressort de commande et de propulsion 62, préalablement comprimé dans la phase précédente, peut se détendre brusquement en renvoyant brutalement le piston de travail 52 de la droite vers la gauche de l'appareil sur la figure 2b. Dans ce mouvement, l'embout 45, lié au piston 52, transmet son déplacement à la tige 44 et, par celle-ci, à la tête du piston de refoulement 40 qui chasse avec une pression multipliée dans le rapport des surfaces de ces pistons le liquide préalablement aspiré dans la chambre de dosage 38. Le volume de liquide ainsi refoulé s'écoule alors par les conduits 28 et 29 jusqu'à la tête d'injection 5, où il repousse la bille 31 à l'encontre de son ressort 32 et s'écoule à travers la microplaquette 36 de la buse 33 qui le projette sous la forme d'un jet mince et très puissant au-delà de la cavité 35. La tête d'injection 5 ayant été préalablement mise par le praticien au contact de l'endroit où doit être réalisée l'injection, le jet ainsi produit pénètre directement dans la muqueuse du patient, selon un processus en lui-même bien connu en technique de mésothérapie. Simultanément, le liquide refoulé de la chambre 38 ne peut revenir vers la carpule 8, la bille 24 du premier clapet étant sous la pression de refoulement appliquée sur l'extrémité du conduit 19 qu'elle obture hermétiquement.

L'appareil selon l'invention est conçu de telle sorte que, consécutivement au ressort de commande et de propulsion 62 et au rappel du piston de travail 52 dans sa position initiale, un dispositif de commande (non représenté) a coupé l'admission de l'air comprimé, qui ne peut être à nouveau fourni à l'appareil que par une nouvelle action de commande du praticien agissant sur la pédale correspondante. L'appareil évite ainsi un fonctionnement séquentiel en continu, qui présenterait le risque grave de produire des injections successives de liquide non contrôlées en particulier avant que la buse ne soit convenablement amenée au droit de la région précise où doit être réalisée chacune de ces injections. En variante, en appuyant sur la pédale au pied pour donner une impulsion, le distributeur peut se déplacer vers la droite par appui du piston 52. Le distributeur reste alors dans cette position un certain temps par différence de pression entre la face gauche et la face droite du distributeur. Dans cette position sont empêchés des à-coups de l'appareil.

La bague coulissante 112 permet par ailleurs au praticien d'assurer un contrôle manuel du fonctionnement de l'appareil n'autorisant la mise en oeuvre de la tête d'injection que dans des conditions exactement définies et en évitant notamment que, par une pression provoquée par inadvertance sur la pédale alors que la tête d'injection n'est pas encore convenablement placée au droit de la gencive dans la région souhaitée, ne puisse se produire aucune injection de liquide. Tant que la bague 112 n'est pas, en effet, amenée dans sa position de butée contre la bague d'arrêt 113, la face inclinée 115 du passage 114 s'appuie sur la tête 116 de la soupape de sécurité 117, mettant de façon permanente le jeu annulaire 92 à l'échappement libre, de telle sorte que l'air comprimé amené par les conduits 103 et 104 dans ce jeu 92 s'échappe directement vers l'extérieur, sans pouvoir exercer sa pression dans la chambre 51 et déplacer le piston de travail 52, armant la tête d'injection. En revanche, une fois la buse 33 exactement amenée au contact de la gencive à l'endroit désiré, le praticien en repoussant la bague 112 met l'appareil en état de fonctionner, toute pression sur la pédale assurant alors, selon le processus décrit, le remplissage de la chambre de dosage puis la formation du jet d'injection par l'effet du brusque rappel dû au ressort de refoulement.

Comme il résulte déjà de la description qui précède, la mise en oeuvre de l'appareil d'injection selon l'invention présente de nombreux avantages. En particulier, l'appareil permet par un simple réglage de la bague filetée 109 montée à l'arrière de la partie moteur 3 du corps 1, de régler à la demande les dimensions de la fente d'échappement 107 de l'air comprimé et par là-même, de créer dans la chambre 119 une contre-pression réglable qui s'oppose au mouvement du piston de travail 52. Selon la nature du liquide à injecter et les conditions de son utilisation, en particulier la profondeur à l'intérieur de la muqueuse dentaire à laquelle ce liquide doit être injecté, le praticien peut ainsi régler de la façon la plus simple et la plus rapide, le fonctionnement de son appareil par simple rotation de la bague 109, celle-ci pouvant d'ailleurs comporter au niveau de sa partie 108 pénétrant dans le logement 107 un index de repérage gradué directement avec des indications caractérisant la force du jet créé et/ou la profondeur d'injection pouvant être réalisée avec celui-ci.

La constitution de la tige 44 en plusieurs éléments de longueurs respectives déterminées destinées à ajuster le volume utile de la chambre de dosage permet de modifier à volonté la quantité de liquide injectée à chaque course du piston de travail à travers la buse d'injection. La présence de la fenêtre 7a prévue au droit du logement 7 dans la partie médicale 2 du corps 1, où peut être également gravée une graduation appropriée permet de contrôler à tout moment la quantité de liquide restant disponible et notamment de prévenir le praticien de l'épuisement prochain de la carpule 8. Le remplacement de celle-ci une fois vide par une autre pleine contenant le même ou un autre liquide peut alors être effectué facilement, par séparation de la partie médicale 2 de la partie moteur 3 après avoir dévissé la bague 4, retrait de la carpule vide et mise en place d'une carpule neuve enfoncée dans le logement sur la pointe du trocart 21.

La forme extérieure de l'appareil est tout spécialement déterminée de telle sorte qu'elle soit pratiquement identique à celle des autres outils d'intervention dont dispose le praticien sur son pupitre de travail; en particulier, le corps 1 peut être facilement tenu à la main à la manière d'un stylo de préférence au niveau de la cassure formée par les deux éléments 2a et 2b de la partie médicale 2, la partie médicale 3 reposant dans le creux formé entre le pouce et l'index, cette prise permettant une grande précision dans le positionnement de la tête d'injection à l'intérieur de la cavité buccale du patient. Grâce à un relief (non représenté), usiné autour du corps 1, on peut donner également à l'appareil une surface extérieure antidérapante, facilitant encore sa prise en main, en évitant les risques que l'appareil ne dévie ou n'échappe lors des opérations d'injection.

Un autre avantage déterminant de l'appareil considéré consiste dans sa parfaire asepsie, en particulier au droit de la tête d'injection 5. Le montage tout autour de celle-ci d'un capuchon protecteur 37 permet notamment d'éviter que les projections éventuelles de sang ou de salive du patient ne viennent polluer l'appareil, ce capuchon 37 étant jetable après chaque intervention sur un patient. Par ailleurs, la conception de l'appareil en deux parties séparables, respectivement une partie médicale et une partie moteur, permet d'assurer pour chaque usage sur un patient différent, une stérilisation préalable efficace de la partie médicale, démontée à cet effet de la partie moteur et placée dans un autoclave ou autre appareil analogue, à rayonnements ultraviolets par exemple, alors que la partie moteur qui n'est jamais en contact avec la bouche du patient peut au contraire être utilisée telle quelle.

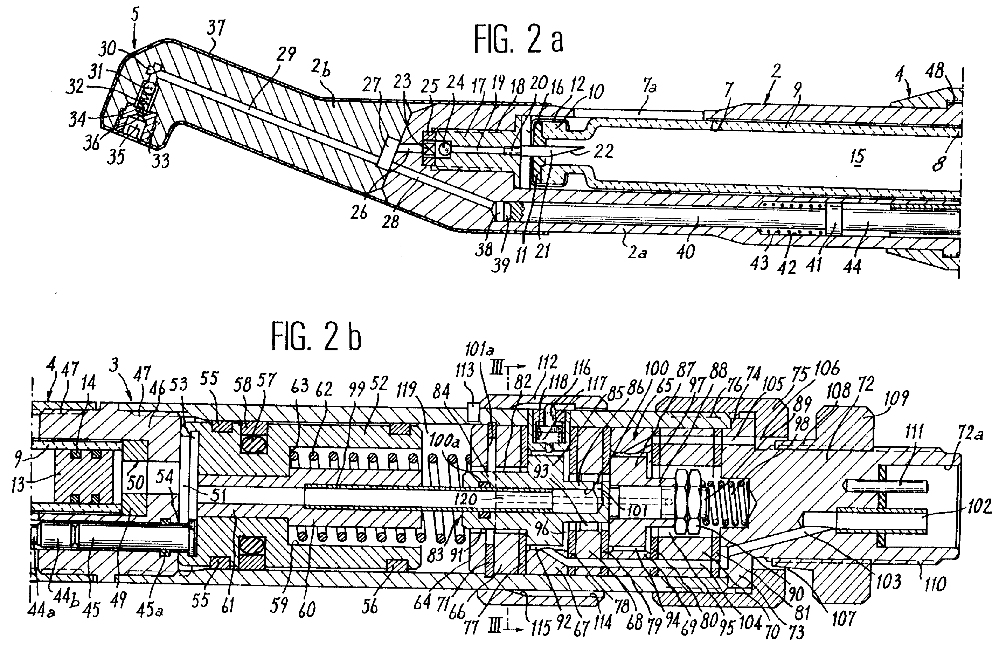

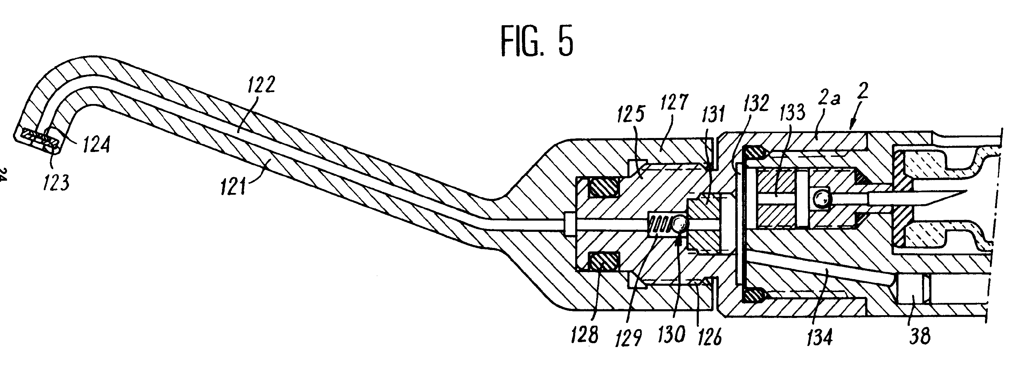

Grâce à la conception de ses diverses parties, l'appareil présente un poids et un emcombrement réduits joints à une facilité d'utilisation tout à fait agréable lui permettant d'amener sans difficulté la tête d'injection bien en contact avec n'importe quelle partie de la muqueuse dentaire à l'endroit où il convient d'effectuer une injection, même dans les positions les plus incommodes, par exemple sur la face linguale des incisives ou au fond de la bouche au voisinage du palais. Le cas échéant, la tête d'injection 5 peut être modifiée pour la rendre encore plus étroite et affinée dans sa partie antérieure pénétrant dans la cavité buccale. Ainsi, elle peut être réalisée de la manière illustrée sur la figure 5, où l'extrémité de l'élément 2b de la partie médicale 2 est ici prolongée par un embout 121 de très faible diamètre, amovible et interchangeable, muni seulement d'un conduit axial 122 qui débouche directement dans une cavité 123 prévue à son extrémité au contact de la zone où doit être réalisée une injection sous pression, et dans le fond de laquelle est monté un injecteur plat 124. Dans cette variante, le clapet qui doit fermer le conduit 122 pour éviter l'échappement du liquide de la carpule pendant la phase de remplissage de la chambre de dosage n'est plus disposé au voisinage de l'injection 124 mais dans l'élément 2a de la partie 2 du corps dans un prolongement 125 de celui-ci sur lequel se visse par un filetage interne 126 un manchon 127 prévu à l'extrémité de l'embout 121, opposée à celle où se trouve l'injecteur 124, l'étanchéité du montage étant réalisée par un joint 128. Bien entendu, d'autres modes de fixation du manchon 127 sur le prolongement 125, pourraient être envisagés, par exemple par cliquet à billes ou verrou à baïonnette, la version de l'appareil ainsi prévu étant plus directement adaptée pour le cas où, par suite de nouvelles normes ou obligations réglementaires, il serait nécessaire de jeter ou de stériliser après usage tout élément ayant pénétré dans la bouche du patient, ceci par souci de sécurité et de protection à la fois des personnes soignées et du praticien.

Le conduit 122 débouche dans une chambre 129 où est disposé un clapet à bille 130 lui-même formant un canal prévu à travers une bague 131 vissée dans un logement correspondant. Dans la partie 2a est par ailleurs ménagé un évidement 132 où débouchent à la fois un conduit 133 relié à la carpule à travers un ensemble analogue à celui déjà décrit en relation avec la figure 2a et un autre conduit 134 reliant l'évidement 132 à la chambre de dosage 38.

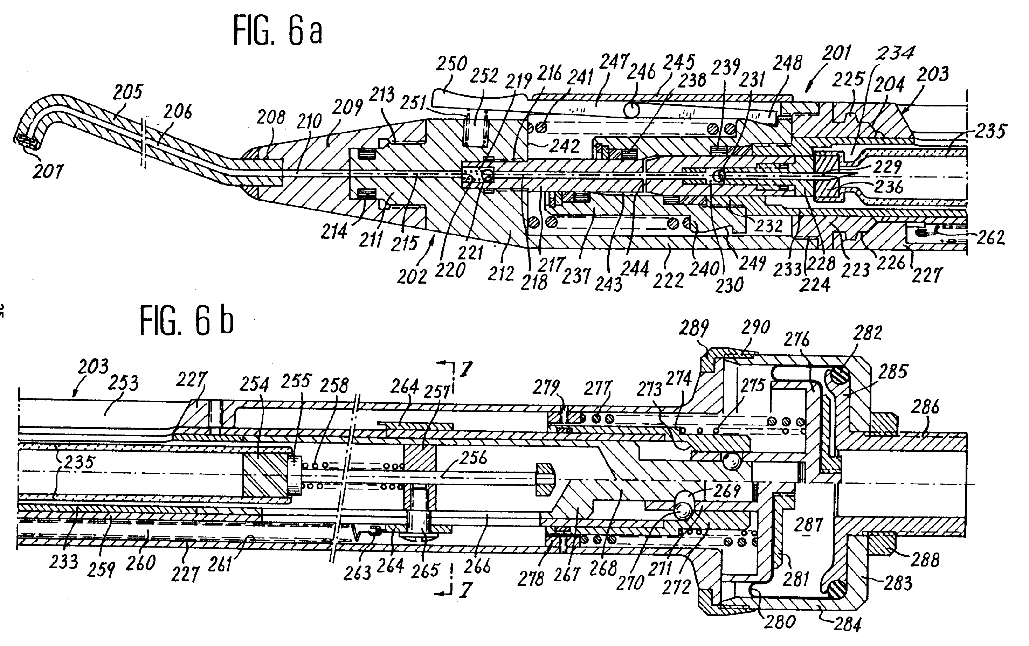

Sur les figures 6a et 6b qui, mises bout à bout, illustrent une section longitudinale d'une autre variante de l'appareil considéré, la référence 201 désigne le corps de celui-ci, constitué comme dans l'exemple précédent de deux parties coaxiales, respectivement 202 et 203, la première dite partie médicale étant susceptible de s'accoupler à la seconde dite moteur au moyen d'un système de liaison rapide 204, du genre quart de tour ou autre.

A son extrémité située à gauche sur le dessin, la partie 202 comporte un bec terminal 205 fin et allongé comportant un canal d'amenée 206 pour un liquide d'injection approprié vers une buse d'extrémité 207. A l'opposé de celle-ci, le bec 205 est emmanché dans un logement 208 prévu en bout d'un support 209 comportant un canal axial 210 disposé dans le prolongement du canal 206. Ce support 209 est lui-même monté dans l'extrémité 211 d'un pièce intermédiaire 212 sur laquelle il est vissé en 213 avec interposition d'un joint d'étanchéité 214. Le canal 210 se prolonge à son tour par un canal 215 dans cette pièce intermédiaire 212 qui comporte par ailleurs un filetage 216 recevant un mandrin 217, lui-même percé axialement d'un conduit 218 dans l'axe du canal 215 et se raccordant à ce dernier par l'intermédiaire d'un clapet à bille 219 soumis à l'action d'un ressort 220 de telle sorte que sous l'effet de ce ressort, une bille 221 obture normalement l'extrémité du conduit 218.

La pièce intermédiaire 212 se prolonge vers la droite sur le dessin par un manchon extérieur 222 entourant le mandrin 217, ce manchon étant solidarisé d'un bouchon d'extrémité 223 vissé en 224. Le bouchon 223 comporte des nervures en saillie 225 propres à coopérer avec des rainures de même profil 226 pour constituer, à l'extrémité d'un manchon de même diamètre 227 prolongeant le manchon 224, le système de liaison rapide 204 entre les deux parties 202 et 203 du corps de l'appareil.

A son extrémité opposée au bec 205, le mandrin 217 comporte un embout de fermeture 228 permettant le montage dans l'axe du conduit 218 d'une aiguille ou trocart 229. Celle-ci peut être normalement fermée par un clapet monté dans une cavité 230 du mandrin et comportant une bille 231, propre à venir obturer l'extrémité correspondante du trocart.

Autour du mandrin 217 est monté coulissante l'extrémité tubulaire 232 d'un manchon creux 233, délimitant intérieurement un logement 234 à l'intérieur duquel est montée une carpule 235 dont l'extrémité dirigée vers le bac 205, comporte un bouchon 236. Celui-ci est préalablement à l'utilisation de l'appareil percé par l'extrémité du trocart 229 afin de permettre, dans des conditions qui seront précisées plus loin, l'écoulement d'un liquide contenu dans cette carpule à travers le trocart 229 puis au-delà de celui-ci, dans le conduit 218 pour autant que la bille 231 libère le passage nécessaire à cet écoulement.

Sur l'extrémité tubulaire 232 du manchon 233 est monté un piston mobile et creux 237, propre à coulisser sur le mandrin 217, des joints annulaires 238 et 239 montés à l'intérieur du piston assurant l'étanchéité. Le piston 237 présente une portée transversale 240 d'appui pour un ressort de commande 241 portant à l'opposé contre une face de butée 242 prévue sur la pièce intermédiaire 212. Le piston mobile 237 délimite ainsi avec le mandrin fixe 217 une chambre annulaire 243 constituant la chambre de dosage du produit soutiré de la carpule 235 et amené dans cette chambre par au moins un passage transversal 244 prévu dans le mandrin.

Sur le manchon externe 222 est rapporté et fixé un capot extérieur 245 sur lequel est articulé autour d'un axe 246 un levier 247, muni à une extrémité d'un talon 248 propre à bloquer en position le piston mobile 237 par engagement dans un logement ouvert 249 de ce piston. A l'opposé du talon 248, le levier 247 comporte une empreinte de commande 250 pour le doigt de l'utilisateur permettant de faire basculer le levier autour de son axe à l'encontre d'un ressort 251 monté dans un logement 252 de la pièce intermédiaire 212, ce basculement faisant ainsi échapper le talon 248 au logement 249.

Le manchon 227 comporte une lumière 253 permettant de visualiser directement la carpule 235, à travers le corps de l'appareil, et en particulier lorsque celle-ci est réalisée en une matière plastique transparente, de suivre l'évolution du niveau du liquide qu'elle contient, au fur et à mesure des injections des doses successives, l'évacuation du liquide s'accompagnant du déplacement d'un fond coulissant 254, à l'opposé du bouchon 236, ce fond 254 étant soumis à l'action de la tête 225 d'une tige de piston 256, montée coulissante à travers un support de guidage 257, en étant soumise à l'action permanente d'un ressort 258.

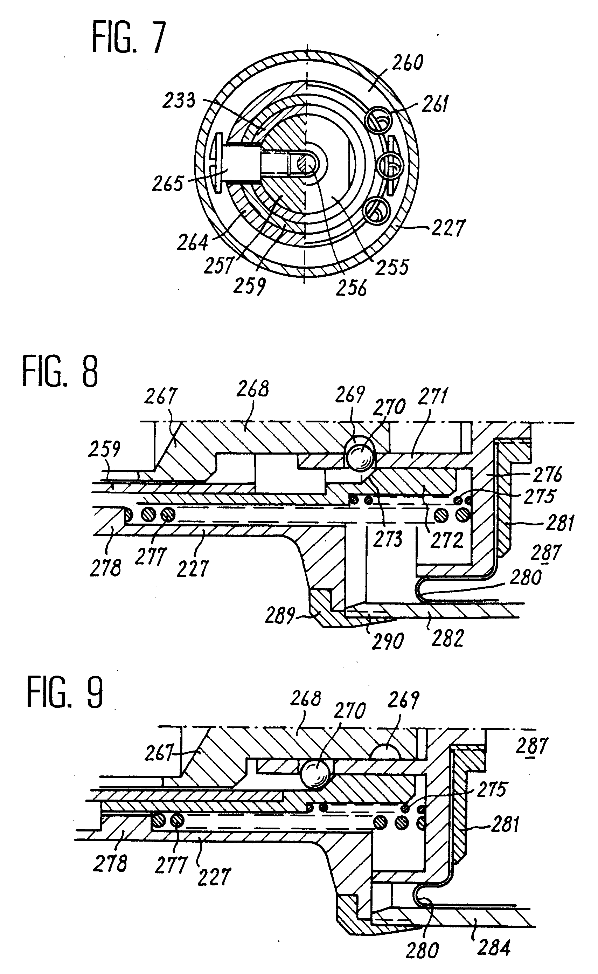

Entre le manchon creux 233 et les manchons extérieurs 222 et 227 est monté un tube de guidage 259, délimitant avec le manchon 227 un espace 260 dans lequel sont montés des ressorts 261, régulièrement répartis autour de l'axe de ce manchon et fixés à leurs extrémités respectivement en 262 sur le manchon 227 et en 263 sur une rondelle cylindrique 264, rendue solidaire par une vis transversale 265 du support du guidage 257 à travers une lumière 266, ménagée latéralement dans le manchon 233 et le tube 259 (figure 7).

A sa partie supérieure, le manchon 233 comporte un embout 267, prolongé axialement par une partie terminale 268, munie de gorges ouvertes 269 propres à recevoir une pluralité de billes 270 montées dans cette gorge afin de bloquer en position cette partie 268 vis-à-vis d'un tube coaxial 271. Le tube de guidage 259 comporte également un prolongement 272, venant coiffer par l'extérieur le tube 271, en immobilisant les billes 270. Le prolongement 272 présente une portée inclinée vers l'intérieur 273 et à l'extérieur un épaulement 274 pour un ressort de rappel 275, en appui à l'opposé contre un rebord 276 formant pièce de poussée axiale, s'étendant transversalement à l'extrémité du tube coaxial 271. Un autre ressort 277 est monté à l'extérieur du tube 259 et s'appuie également contre le rebord 276, celui-ci étant arrêté à l'opposé par une rondelle 278 bloquée par des vis 279 contre la surface interne du manchon 227.

Le rebord 276 est associé à une membrane d'étanchéité 280 immobilisée contre la face supérieure du rebord par une plaquette d'appui 281, cette membrane étant terminée par un bourrelet 282, emprisonné entre le fond 283 d'un bouchon terminal 284 prévu en bout de l'appareil et une pièce de serrage 285 munie d'un prolongement tubulaire 286 permettant d'introduire dans le corps de l'appareil derrière la membrane 280, une pression convenable d'air comprimé dans une chambre 287, ainsi délimitée. Le bouchon terminal 284 est immobilisé sur le prolongement tubulaire 286 au moyen d'un écrou 288 et est également maintenu par une bague 289 solidaire du manchon 227 dans laquelle est vissé le bouchon 284 grâce à un filetage interne 290.

Les figures 8 et 9 permettent, en liaison avec les figures 6a et 6b, de mieux illustrer la manière dont est mis en oeuvre l'appareil dans le mode de réalisation décrit ci-dessus, où l'ensemble constitué par les pièces 237, 233, 267, 268 constitue notamment le piston de travail, le rebord 276, le tube 271 et la membrane 280, constituant la pièce de poussée.

Pour pouvoir notamment procéder à une injection d'une dose donnée de liquide à travers la buse 207 préalablement positionnée au voisinage de l'endroit où doit être réalisée cette injection, l'utilisateur, dans un premier temps, libère par le levier 247, le piston 237, en dégageant le talon 248 de ce levier vis-à-vis du logement 249. A cet instant et par une commande appropriée, par exemple au pied, il introduit par le prolongement tubulaire 286, une pression d'air comprimé convenable dans la chambre de compression 287. Sous l'effet de celle-ci, l'ensemble formé par le rebord 276 et le tube 271 se déplace en entraînant simultanément par la partie terminale 268 le manchon 233 lui-même provoquant le déplacement du piston 237 à l'encontre du ressort 241.

Dans ce mouvement, la chambre de dosage 243 est progressivement remplie de liquide aspiré hors de la carpule 235 par le trocart 229, le conduit 218 et les passages transversaux 244, l'écoulement du liquide étant autorisé par le déplacement de la bille 231 dans le logement 230 qui libère l'extrémité ouverte du trocart.

Ce mouvement se poursuit notamment jusqu'à ce que les billes 270, qui solidarisent le tube 271 de l'embout terminal 268 du piston moteur se présentent devant la portée inclinée 273 du prolongement 272, en permettant alors l'échappement de ces billes hors de leur logement 269. A cet instant, le piston de travail est brutalement libéré, le ressort 241 repoussant brutalement l'ensemble mobile en sens inverse. Simultanément, le liquide contenu dans la chambre de dosage 243 est expulsé hors de celle-ci par les passages 244 et le conduit 218 sous l'effet de la pression, la bille 221 fermant normalement le conduit 218 étant repoussée à l'encontre de son ressort 219, en permettant l'échappement du liquide vers les conduits successifs 215, 210 et 206 jusqu'à la buse d'injection 207. En même temps, la bille 231 revient fermer l'extrémité du trocart 229, en évitant le refoulement du liquide vers la carpule.

Dans la phase terminale du mouvement, les ressorts 274 et 277 ramènent le rebord 276 en position initiale avec retour des billes 270 dans leur logement 269 pour solidariser à nouveau le tube 271 et la partie terminale 268, l'appareil étant prêt pour un nouveau cycle de fonctionnement.

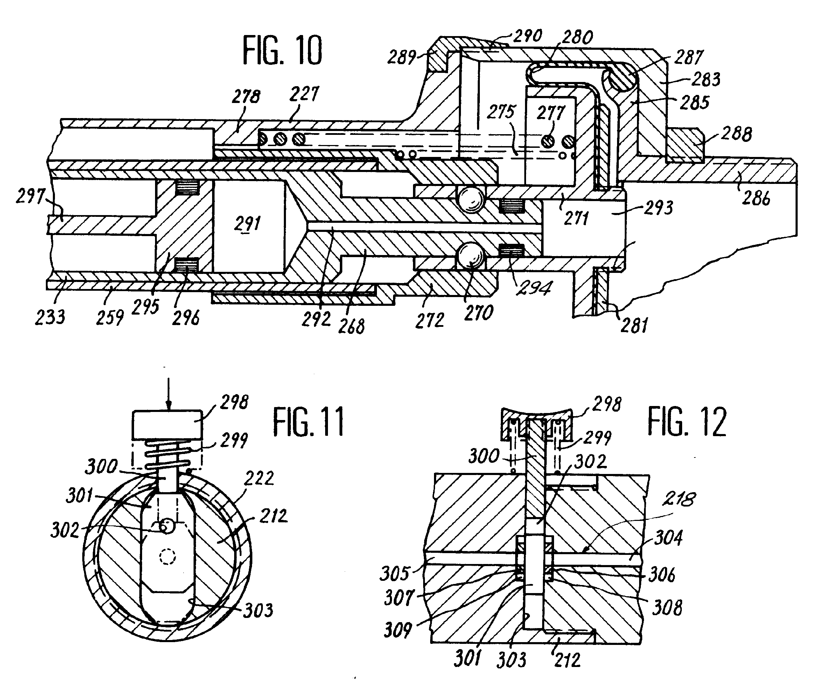

La figure 10 illustre une variante de réalisation de l'appareil représenté sur les figures 6a et 6b, en ce qui concerne les moyens permettant d'exercer sur le fond 254 de la carpule 235 une pression convenable, quel que soit le degré de remplissage de cette carpule au fur et à mesure des cycles de fonctionnement de l'appareil, avec formation à chaque tir à travers l'injecteur 207, d'un jet de liquide fin et sous pression.

Dans cette variante, le prolongement 268 du manchon creux 233 qui coulisse dans le tube 271 délimite du côté de la carpule une chambre étanche 291, réunie par un conduit axial 292 traversant le prolongement 268 à la partie opposée du tube 271, elle-même en communication par le prolongement tubulaire 286 avec la source extérieure d'air comprimé provoquant, de la façon décrite ci-dessus, le déplacement de la pièce de poussée. Un joint annulaire d'étanchéité 294 est porté par le prolongement 268 et est en contact avec la surface interne du prolongement 271. Dans la chambre 291 est monté un piston 295, portant lui-même un joint annulaire d'étanchéité 296 en contact avec la surface interne du manchon creux 233, 268, ce piston 295 étant relié par un axe 297 au fond 254 de la carpule. Dans cette variante, c'est donc directement la pression de l'air dans la chambre 291, s'exerçant sur le piston 295 qui est en permanence transmise au fond 254, en transmettant au liquide de la carpule une poussée qui se maintient constante à elle-même à chaque fois que la pression d'air de commande est admise dans l'appareil. Ce dispositif est ainsi équivalent à celui décrit en relation avec les figures 6a et 6b, mettant en jeu les ressorts 261 et le support de guidage 257.

Dans le premier mode de réalisation envisagé précédemment, l'appareil ne peut être mis en oeuvre que dans la mesure où l'utilisateur a préalablement fait basculer le levier de blocage 247, en exerçant sur l'empreinte de commande 250 un effort limité à l'encontre du ressort 252, en dégageant ainsi le talon 248 du levier et en libérant le manchon creux 233.

Les figures 11 et 12 illustrent une autre variante de réalisation qui, sans viser à bloquer cette fois le manchon creux, empêche néanmoins l'échappement du liquide hors de la chambre annulaire 243, ne permettant donc à nouveau mais sous une autre forme, la création du jet à la sortie de l'injecteur qu'une fois l'appareil préalablement et convenablement positionné, l'utilisateur autorisant alors seulement la mise en oeuvre du cycle de fonctionnement.

Dans cette variante, le dispositif d'interdiction provisoire du fonctionnement est constitué par un bouton 298, normalement repoussé vers le haut par rapport au corps 222 grâce à un ressort 299, ce bouton comportant une tige 300 solidarisée d'une plaquette transversale 301 et munie d'un trou central 302. La plaquette est montée à coulissement vertical sous l'effet du bouton 298 à l'intérieur d'un logement 303 prévu dans la pièce intermédiaire 212. Comme on le conçoit aisément à la vue des figures 11 et 12, lorsque le bouton 398 est en position relâchée, la plaquette transversale 301 obture la communication entre les deux parties 304 et 305, respectivement situées de part et d'autre du conduit 218, des joints d'étanchéité 306 et 307 étant avantageusement prévus de chaque côté dans des chambrages 308 et 309 les recevant en regard de la plaquette dans le logement 303. En revanche, si l'utilisateur exerce sur le bouton 298 un effort dirigé vers le bas à l'encontre du ressort 299, il amène le trou 302 en coïncidence avec les deux parties 304 et 305, en assurant ainsi la continuité du conduit 218 avec écoulement du liquide soutiré de la chambre 243 et refoulement sous pression de celui-ci vers l'injecteur 207.

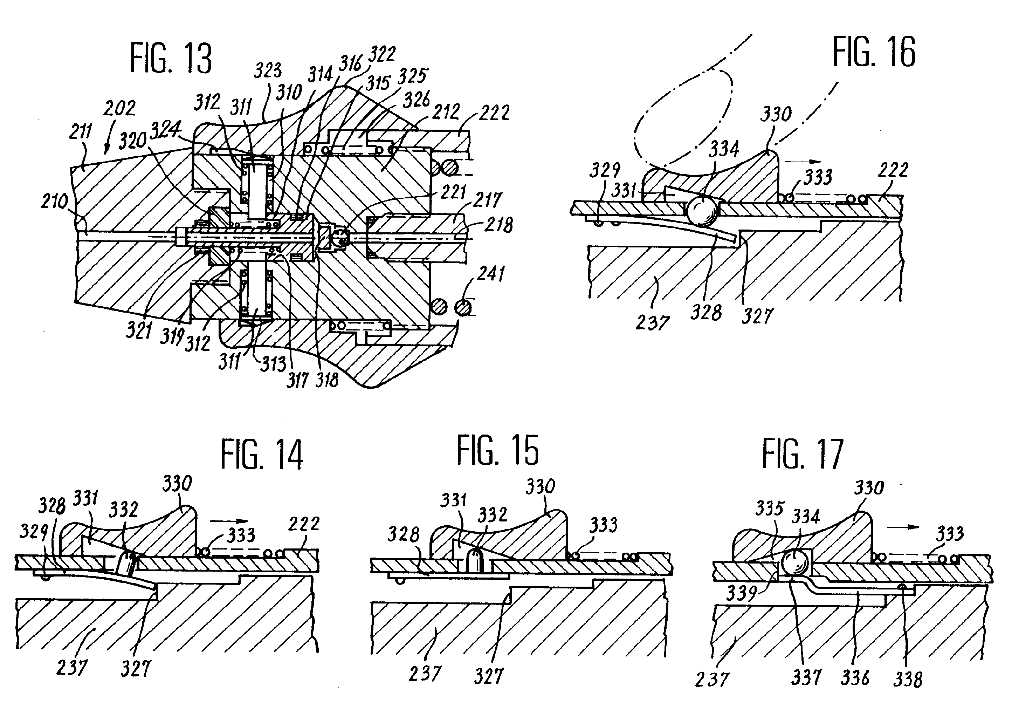

Les figures 13 à 17 illustrent d'autres variantes des moyens propres à réaliser, sous d'autres formes, l'interdiction provisoire du fonctionnement de l'appareil, tant que celui-ci n'est pas amené en position convenable, avec l'injecteur 207 à proximité immédiate de l'endroit où doit être réalisée le jet de liquide.

Dans l'exemple illustré sur la figure 13, la pièce intermédiaire 212 comporte un trou transversal 310 pour le montage de pions radiaux 311, soumis chacun à l'action d'un ressort 312 en appui contre une tête débordante 313 et tendant à repousser ces pions vers l'extérieur de la fente, en dégageant le talon 314 d'un plongeur 315 muni d'un joint d'étanchéité 316 dans sa surface externe et montée coulissante dans un logement 317 de la pièce intermédiaire 212. Le plongeur 315 comporte un téton d'extrémité 318 propre à bloquer en position de fermeture la bille 221 du clapet qui obture ainsi l'extrémité du conduit 218. Ce même plongeur est soumis à l'action d'un ressort 319 en appui sur une plaquette fixe 320 montée dans la partie en regard de la pièce d'extrémité 211 avec interposition d'un joint d'étanchéité 321.

Dans la variante considérée, la pièce intermédiaire 212 est associée à une molette coulissante 322, comportant une empreinte externe 328 permettant à l'utilisateur de la faire librement coulisser sur la surface externe de la pièce en amenant en regard des têtes 313 des pions 311 un évidement annulaire 324 permettant ainsi à ces pions de s'échapper vers l'extérieur, en libérant le talon 314 et par suite le plongeur 315 et la bille 221. La molette coulissante 322 est en permanence rappelée en position de verrouillage des pions 311 grâce à un ressort 325 monté dans un logement 326 entre le manchon extérieur 222 et une portée d'appui de cette molette de telle sorte que si l'opérateur relâche son effort à l'encontre de ce ressort, celui-ci remène la molette dans la position où les têtes des pions 311 sont rappelées vers l'intérieur en bloquant à nouveau le plongeur 315.

Dans la variante illustré sur les figures 14 et 15, un montage sensiblement équivalent mais plus simple est représenté, le piston 237 comportant un épaulement externe 327 formant butée pour l'extrémité d'une lame de ressort 328, immobilisée par un rivet 329 sur une molette coulissante 330. Cette molette 330 comporte un évidement interne 331 dans lequel peut se débattre, selon la position axiale de la molette sur le manchon extérieur 222, un téton 332 prévu en bout de la lame 328. Un ressort 333 est monté derrière la molette pour assurer le rappel en position de celle-ci.

Sur la figure 14, la molette 330 est telle que la partie étroite du logement 331 repousse le téton 332 vers l'intérieur, la lame 328 bloquant dans ces conditions le piston 237 et par suite l'ensemble des éléments qui constituent la pièce de poussée de l'appareil, du fait de l'appui de l'épaulement 327 sur la lame 328.

Dans la position illustrée sur la figure 15, la molette 330 est repoussée à l'encontre du ressort 333, en permettant aux pions 332 l'échappement dans la partie plus large de l'évidement 331, la lame de ressort 328 sortant de l'épaulement 327 en libérant ainsi le piston 237.

Sur la figure 16, la variante illustrée est équivalents à celle envisagée ci-dessus, le téton 332 étant ici remplacé par une bille 334, facilitant le déplacement de la molette en refoulant dans le logement 331.

Sur la figure 17 enfin, on a repris une disposition analogue mais dans laquelle la bille 334 peut s'effacer dans un évidement 335 de la molette 330 qui présente un profil inverse de celi prévu dans les exemples précédents, la lame de ressort 336 étant cette fois fixée par un rivet 338 sur la pièce intermédiaire 212, cette lame étant conformée de telle sorte que son extrémité libre 337 soit coudée pour venir en butée contre une portée 339, prévue dans le manchon extérieur 222.

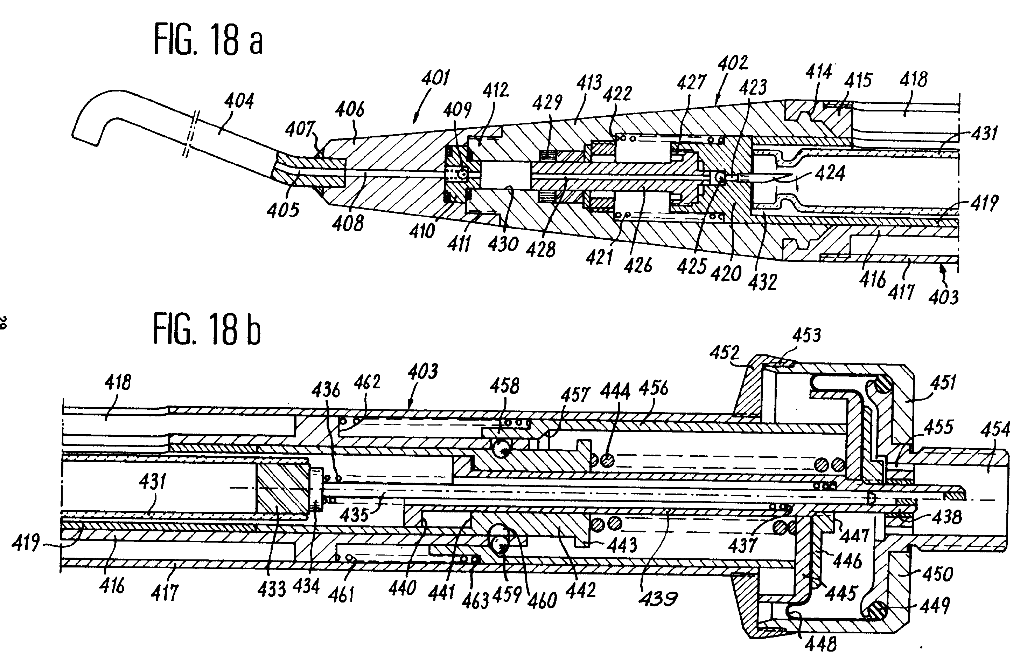

Les figures 18a et 18b illustrent une autre variante de réalisation de l'appareil selon l'invention, agencée de telle sorte que, à la différence de la première variante où le liquide est soutiré de la carpule dans la chambre de dosage annulaire lorsque la pièce de poussée revient en position arrière, ce soutirage s'effectue ici lors du recul du mandrin 426 par l'intermédiaire du ressort de rappel 421, avant que la pièce de poussée ne soit brutalement libérée pour produire, à travers l'injecteur d'extrémité, le jet désiré.

Dans cette variante, l'appareil comporte comme précédemment un corps 401 formé de deux parties coaxiales, respectivement 402 et 403. La partie intérieure 402 comport un bec terminal 404 supportant en bout l'injecteur (non représenté) et muni d'un conduit interne 405. Il se raccorde à une partie d'extrémité 406 par un moyen de liaison approprié 407. Dans cette pièce 406 est prévu un conduit 408, pourvu d'un clapet à bille 409 monté dans une pièce de support 410. La pièce 406 est réunie par un filetage 411 avec l'extrémité 412 d'une pièce intermédiaire 413. A l'opposé, cette dernière est munie d'un organe d'accouplement rapide 414 avec un raccord 415 prévu en bout d'un tube 416 lui-même doublé par un manchon externe 417. Une fenêtre latérale 418 est prévue dans ce manchon de manière analogue aux dispositions déjà envisagées dans la première variante.

A l'intérieur du tube 416 est monté un manchon creux 419, terminé à son extrémité dirigée vers le bec 404, par un piston 420. Un ressort de rappel 421 est prévu entre ce piston 420 et une portée d'appui 422 prévue dans la pièce intermédiaire 413. A l'intérieur du piston 420 est ménagé un conduit 423, prolongé d'un côté par une aiguille ou trocart 424 et obturé à l'autre par un clapet à bille 425, prévu en regard d'un mandrin 426 vissé dans le manchon 420 en 427 et comportant un conduit axial 428, ce mandrin pouvant se déplacer au contact d'un joint d'étanchéité 429 monté dans la surface interne d'un logement 430 prévu dans la pièce 413.

L'aiguille 424 perfore le bouchon d'extrémité d'une carpule 431 montée dans la région interne 432 délimitée entre le manchon creux 419 et le piston 420.

A son extrémité opposée, la carpule 431 est fermée par un fond mobile 433, soumis en permanence à la poussée d'un piston 434 solidaire d'une tige axiale 435, un ressort 436 étant monté entre le piston 434 et une portée d'appui 437 du prolongement cylindrique 438 d'un tube 439.

Ce tube 439 comporte un épaulement 440 monté mobile à l'intérieur du manchon creux 419 et susceptible de venir en appui contre un rebord interne 441 prévu dans un embout 442 formé à l'extrémité de ce manchon. L'embout 42 présente un épaulement 443 formant portée d'appui pour un fort ressort 444, lui-même appliqué à son extrémité opposée sur un rebord 445 immobilisé sur le prolongement 438 du tube 439 par une bague 446, celle-ci étant vissée sur un filetage 447 dans la surface externe du prolongement 438. Une membrane élastique 448 est immobilisée entre le rebord 445 et la bague 446, cette membrane présentant un bourrelet d'extrémité 449 maintenu entre une bride 450 et un couvercle 451. Celui-ci est enfin immobilisé vis-à-vis du manchon 417 par une bague 452 vissée en 453.

Sur la figure 18b, on a figuré en 454 l'arrivée d'air comprimé dans l'appareil, celui-ci pouvant traverser le couvercle 451 à travers des orifices de passage 455 pour venir exercer sur le rebord 445 un effort approprié. L'étanchéité au cours du déplacement de la pièce de poussée de l'appareil est réalisée par la membrane 448. Le rebord 445 se prolonge par une pièce tubulaire 456, mobile coaxialement dans le manchon extérieur 417. Cette pièce 456 comporte dans sa surface interne une portée inclinée 457 et un prolongement cylindrique 458, coulissant au contact de la surface externe du manchon creux 416 au contact de billes de blocage 459 emprisonnées dans des logements 460 prévus dans l'embout d'extrémité 442 du manchon 419. Un ressort 461 est monté entre une portée d'appui fixe 462 du tube 416 et un épaulement 463 de la pièce tubulaire 456.

Dans cette troisième variante de réalisation, le fonctionnement de l'appareil est le suivant : lorsque l'utilisateur a amené l'appareil au voisinage précis de l'endroit où doit être effectuée une injection de liquide l'appareil étant muni d'un dispositif de verrouillage analogue à l'un de ceux décrits précédemment dans l'une quelconque des figures 6 à 17 mais ici non représenté, il commande par un contacteur au pied par exemple, l'admission d'air qui par les orifices 455 vient exercer sur le rebord 445 formant piston une poussée correspondante, en comprimant dans un premier temps le ressort de commande 444. Dans ce mouvement, le tube 439 se déplace avec le rebord, en exerçant un effort sur le piston 434 et le fond 433 de la carpule 431 par le ressort 436. La pression correspondante dégage la bille 425 du conduit 423 et permet au liquide de la carpule de venir remplir le logement 430 formant chambre de dosage, le mandrin 426 restant en position fixe du fait de la poussée en sens inverse du ressort 421.

Simultanément, la pièce tubulaire 456 se déplace avec le rebord 445 en comprimant le ressort 461.

En fin de course, la pièce tubulaire 456 présente sa portée inclinée 457 en regard des billes 456 et permet à ces dernières de s'échapper vers l'extérieur, entre l'embout 442 et la pièce tubulaire. L'embout 442 et par suite le manchon creux 419 dont il est solidaire sont brutalement libérés, le ressort 444 préalablement comprimé par le mouvement du rebord 445 se détendant brutalement en repoussant ce manchon 419 et avec celui-ci le piston 420 et le mandrin 426. Le clapet 425 se referme tandis que le clapet 409 s'ouvre en permettant au liquide accumulé dans le logement 430, d'être totalement et brusquement expulsé par les conduits 408 et 405 jusqu'à l'injecteur prévu à l'extrémité du bec 404.

Dans la phase suivante, la pression d'air étant relâchée, les ressorts 421 et 461 ramènent l'ensemble des pièces mobiles en position initiale, la butée du rebord 445 contre la bride 450 permettant à l'embout 442 de se déplacer seul en fin de course, jusqu'à ramener les billes 459 en regard de leurs logements 460 dans lesquels elles sont alors repoussées, en verrouillant à nouveau l'embout et le tube 416 jusqu'à l'engagement d'un nouveau cycle de fonctionnement.

Bien entendu et comme il résulte déjà de ce qui précède, il va de soi que l'invention ne se limite pas aux seuls exemples de réalisation plus spécialement envisagés ci-dessus; elle en embrasse au contraire toutes les variantes. En particulier, on pourrait sans modifier la cinématique de fonctionnement de l'appareil et ses caractéristiques éventuelles décrites précédemment, prévoir par exemple dans la partie médicale, deux voire trois chambres de dosage disposées parallèlement dans le corps, chacune de celle-ci étant associée à un piston de refoulement actionné simultanément avec les autres par le piston de travail, afin de produire au niveau de la tête, munie d'autant d'injecteurs voisins, deux ou trois jets parallèles, propres à pénétrer en même temps la muqueuse à traiter. De même, dans d'autres variantes, on pourrait prévoir d'associer à une seule chambre de dosage et un seul injecteur réuni à celle-ci, deux logements dans le corps, contenant chacun une carpule d'un liquide différent, avec un inverseur permettant de réunir l'un ou l'autre à la chambre de dosage, ceci afin de réaliser successivement des injections de ces liquides sans avoir à démonter l'appareil et à changer la carpule qu'il contient. Enfin, on peut prévoir sur le pupitre de travail du praticien, un ensemble de contrôle qui informe celui-ci à tout moment sur la pression d'injection, le nombre d'injection réalisé, le volume de celles-ci, etc...

## Revendications

1. Appareil d'injection de liquide sans aiguille, comportant un corps allongé (1 - 201 - 401) en deux parties séparables placées bout à bout, dont la première dite médicale (2 - 202 - 402), comprend au moins un logement (7 - 234 - 432) recevant une carpule étanche (8 - 235 - 431) contenant le liquide à injecter et au moins une chambre de dosage (38 - 243 - 430) à volume variable, ménagée dans ladite première partie et communiquant avec une buse (33 - 207) munie d'un injecteur propre à former un jet de liquide étroit consécutivement à un brutal refoulement du liquide hors de la chambre de dosage, et dont la seconde partie (3 - 203 - 403), dite moteur, comporte un ensemble mobile à mouvement alternatif comprenant un piston de travail (52 - 233 - 420) et un ressort de commande et de propulsion (62 - 241 - 444), agissant sur ce piston en étant progressivement comprimé sous l'effet du déplacement d'une pièce de poussée (52 - 276 - 445), provoqué par une pression d'un fluide de propulsion dans une chambre étanche (51 - 287) prévue dans le corps, caractérisé en ce que la carpule présente un fond mobile dont le déplacement réalise l'envoi de liquide depuis cette carpule vers la chambre de dosage, en ce que le fluide de propulsion est de l'air comprimé et en ce que le déplacement continu de la pièce de poussée se produit jusqu'au moment où le ressort est automatiquement et brutalement libéré au moyen d'un organe mobile (83 - 270, 271, 272 - 458, 459, 460), à déplacement limité, autorisant le retour rapide du piston simultanément avec l'expulsion du liquide hors de la chambre de dosage, préalablement au retour en position initiale de la pièce de poussée.

2. Appareil selon la revendication 1, caractérisé en ce que l'ensemble mobile comprend un piston de travail faisant également office de pièce de poussée, le piston (52) séparant l'intérieur du corps (1) dans ladite seconde partie en deux chambres (51 - 119), disposées de part et d'autre dudit piston de travail et dont la première est délimitée partiellement par le fond mobile (13) de la carpule et est progressivement mise sous pression d'air comprimé de manière à déplacer le piston de travail en sens inverse à l'encontre d'un ressort de propulsion (62) logé dans la seconde chambre, ledit ressort provoquant le retour rapide du piston de travail suite à une soudaine réduction de la pression dans ladite première chambre commandée par un distributeur (83) coaxial au piston de travail et déplacé sur une très faible distance par ledit piston en fin de course, de façon à mettre la pression dans ladite première chambre à l'échappement à l'air libre, le piston de travail dans son retour rapide transmettant son mouvement au piston d'expulsion qui chasse le liquide hors de la chambre de dosage vers l'injecteur de la buse.

3. Appareil selon la revendication 1, caractérisé en ce que le piston d'expulsion (40) du liquide hors de la chambre de dosage (38) comporte une tête, munie d'un organe d'étanchéité (39) et montée coulissante dans ladite chambre, ladite tête étant prolongée par une tige (44), éventuellement en plusieurs éléments successifs pour ajuster la longueur de ladite tige, ledit piston d'expulsion (40) étant soumis à l'effet permanent d'un ressort auxiliaire (43) qui repousse la tige contre un embout coaxial (45), solidaire du piston de travail (52) et qui est en contact avec l'extrémité de la tige lorsque les deux parties (2 - 3) du corps sont assemblées.

4. Appareil selon l'une des revendications 1 à 3, caractérisé en ce que le fond du logement (7) de la première partie (2) du corps (1) recevant la carpule (8) comporte un trocart (21), apte à traverser une rondelle souple d'étanchéité (11) fermant ladite carpule pour autoriser la sortie du liquide refoulé par le fond mobile (13) de la carpule déplacé par l'air comprimé admis dans la première chambre (51) de la seconde partie (3) du corps.

5. Appareil selon la revendication 4, caractérisé en ce que le trocart (21) est monté à l'extrémité d'un conduit dont l'extrémité opposée est obturée par un clapet à bille (24), évitant le retour du liquide dans la carpule (8) lors du refoulement du liquide hors de la chambre de dosage (38).

6. Appareil selon l'une quelconque des revendications 1 à 5, caractérisé en ce que la chambre de dosage (38) est reliée à la tête d'injection (5) par un conduit (28 - 29) à l'extrémité duquel est monté un autre clapet à bille (31), soumis à l'action d'un ressort (32), l'ouverture dudit clapet (31) étant provoquée par la pression du liquide chassé de la chambre de dosage.

7. Appareil selon la revendication 6, caractérisé en ce que la tête d'injection (5) comporte au droit et en aval du clapet à bille (31), un support pour une buse amovible (33) portant un injecteur (36), en matériau très dur et résistant à l'abrasion, fournissant le jet étroit sous pression à la sortie de la tête.

8. Appareil selon l'une quelconque des revendications 1 à 5, caractérisé en ce que la chambre de dosage est directement reliée par un conduit (122) prévu dans un embout allongé de très faible diamètre (121) à un injecteur plat (124), disposé au plus près de la surface extérieure de la tête d'injection (5).

9. Appareil selon la revendication 1, caractérisé en ce que les deux parties (2 et 3) du corps (1) sont réunies par une bague filetée (4), portée et arrêtée par l'une des parties (2) et coopérant avec un pas de vis ménagé en bout d'une pièce de fermeture (46), obturant l'extrémité en regard de l'autre partie (3).

10. Appareil selon l'une quelconque des revendications 3 à 9, caractérisé en ce que le piston de travail (52) comporte dans sa face dirigée vers la première partie (2) du corps (1), une rainure interne (53) recevant la tête (54) de l'embout (45), agissant sur la tige (44) prolongeant le piston d'expulsion (40).

11. Appareil selon l'une quelconque des revendications 1 à 10, caractérisé en ce que le distributeur (83) présente un profil cylindrique étagé (84, 85, 86, 87, 88) et est monté mobile axialement et avec jeux annulaires dans un passage (82) prévu dans un bloc-support (65), solidarisé de la paroi interne de la seconde partie (3) du corps (1), par des moyens (66, 73, 75) d'arrêt et de blocage, ledit distributeur délimitant avec des portées d'appui transversales de joints plats (77, 78, 79, 80) montés dans le bloc-support, des jeux radiaux, respectivement (96, 97) et (96a, 97a) permettant alternativement, selon la portion axiale du distributeur, la mise en pression de la première chambre (51) ou l'échappement à l'air libre de celle-ci.

12. Appareil selon la revendication 11, caractérisé en ce que l'échappement à l'air libre de la pression dans la première chambre (51) s'effectue à travers une fente réglable (107) dont la dimension est ajustée à la demande, pour jouer sur la pression du jet de liquide à injecter, au moyen d'une bague (109), propre à être vissée sur un prolongement (72) du bloc-support (65), ladite bague comportant une partie (108) de forme conique pénétrant partiellement dans une cavité (106) délimitée entre ledit prolongement (72) et une seconde bague (75), vissée en bout de la seconde partie (3) du corps (1).

13. Appareil selon l'une des revendications 11 ou 12, caractérisé en ce que l'air comprimé est admis dans la première chambre (51) entre le piston de travail (52) et la pièce de fermeture (46) par un conduit coaxial (99) coulissant dans un alésage (100) du distributeur (83) et réuni au passage (82) prévu dans le bloc-support (65) par un passage radial (101), ménagé à travers le distributeur.

14. Appareil selon l'une quelconque des revendications 11 à 13, caractérisé en ce que le passage (82) prévu entre le distributeur (83) et le bloc-support (65) est susceptible d'être mis à l'air libre par une soupape de sécurité (116), manoeuvrée par une bague coulissante (112) montée sur l'extérieur de la partie moteur (3) du corps (1), ladite bague comportant un évidement interne (114) terminé par une face inclinée (115) de telle sorte que, selon sa position axiale, elle ouvre ou laisse fermée ladite soupape (116), elle-même appliquée contre un siège d'étanchéité par un ressort (118).

15. Appareil selon la revendication 11, caractérisé en ce que le distributeur (83) est soumis à l'action d'un ressort de rappel (98), appliquant ledit distributeur contre les joints plats (77 et 79) qui ferment l'échappement de la pression d'air dans la première chambre (51) tant que le piston de travail (52) ne s'est pas suffisamment déplacé pour repousser ledit distributeur à l'encontre dudit ressort de rappel.

16. Appareil selon l'une quelconque des revendications 1 à 15, caractérisé en ce qu'il est relié à un poste de distribution d'air comprimé commandé par un organe d'actionnement délivrant cet air dans la première chambre (51) jusqu'au déplacement du distributeur mobile (83) commandé en fin de course par le piston de travail (52) et provoquant l'arrêt de l'admission d'air dans ladite chambre, une nouvelle admission d'air pour l'accomplissement du cycle suivant exigeant une répétition de la commande dudit organe.

17. Appareil d'injection de liquide selon la revendication 1, caractérisé en ce que l'ensemble mobile monté dans la seconde partie (203) du corps (201) comporte un piston de travail (237) soumis à l'action d'un ressort de commande (241) progressivement comprimé par une pièce de poussée axiale (276) réunie par une membrane élastique (280) à la paroi interne d'une chambre de compression (287) reliée à une source d'air comprimé, et des moyens pour solidariser le piston de travail avec un organe d'immobilisation temporaire (269 - 270) prévu dans le corps, coaxialement au piston, de telle sorte que, au-delà d'une course déterminée de la pièce de poussée, provoquant la compression progressive du ressort, celui-ci soit brutalement libéré et détermine le déplacement rapide du piston, en réalisant l'expulsion hors d'une chambre de dosage (243) du liquide à injecter, communiquant avec une carpule (235) formant réserve de liquide.

18. Appareil d'injection selon la revendication 17, caractérisé en ce que les moyens de blocage sont constitués par des billes (270) montées dans des logements ouverts (269) prévus dans la paroi externe du piston (237) et maintenus en postion par un manchon coaxial (272), coulissant sur cette paroi sous l'effet de la pièce de poussée, ce manchon comportant un évidement profilé (273) pour l'échappement des billes et la libération du piston au-delà d'une course donnée du manchon.

19. Appareil d'injection selon l'une des revendications 17 ou 18, caractérisé en ce que le piston de travail (237) comporte un manchon coaxial (233) et est monté coulissant par rapport à un mandrin axial (217) fixe, délimitant une chambre annulaire (243) de dosage du liquide entre le mandrin et le manchon, cette chambre communiquant avec un canal axial (218) prévu dans le mandrin dans le prolongement d'un bec (205) muni en bout d'un injecteur de liquide (207) formant un jet étroit sous l'effet de la pression créée par le déplacement du piston de travail (237).

20. Appareil d'injection selon la revendication 19, caractérisé en ce que le canal axial comporte un clapet à billes (231) prévu en regard d'un trocart (229) communiquant avec l'intérieur de la carpule (235) pour autoriser l'admission du liquide dans la chambre de dosage (243) par des conduits radiaux (244) ménagés dans le mandrin (217), ledit clapet obturant la communication avec la carpule lors du déplacement du piston de travail (237) sous l'effet du ressort de commande (241).

21. Appareil d'injection selon l'une quelconque des revendications 17 à 20, caractérisé en ce que le manchon (233) coaxial au piston de travail coopère avec un levier de blocage (247) à commande manuelle articulé autour d'un axe (246) sur le corps (201), ce levier comportant un talon (248) propre à s'engager dans un logement ouvert (249) du manchon (233) pour empêcher son coulissement par rapport au mandrin fixe (217).

22. Appareil d'injection selon l'une quelconque des revendications 17 à 21, caractérisé en ce que la carpule comporte un fond mobile (254) sous l'effet d'un piston d'appui (255) soumis en permanence à l'action de ressorts (260) montés autour d'un manchon creux extérieur (259) dans lequel coulisse le piston de travail, ces ressorts étant solidarisés à l'extrémité d'une traverse (257) se déplaçant dans une lumière (266) du manchon.

23. Appareil d'injection selon la revendication 18, caractérisé en ce que le piston de travail (237) comporte un prolongement tubulaire (268) mobile axialement dans le manchon (272).

24. Appareil d'injection selon la revendication 19, caractérisé en ce que le corps (201) comporte une fente (303) pour le déplacement d'une plaquette transversale (300) actionnée par un bouton-poussoir (298), cette plaquette comportant un passage central (302) permettant d'assurer la continuité du canal (218) pour l'échappement du liquide provenant de la chambre de dosage vers l'injecteur, uniquement lorsque le bouton-poussoir est appuyé à l'intérieur de la fente du corps.

25. Appareil d'injection selon la revendication 17, caractérisé en ce que l'organe d'immobilisation temporaire du piston de travail comporte une molette coulissant sur la surface extérieure du corps (201) pour immobiliser ou libérer les têtes (313) de pions radiaux (311) par rapport à un évidement (324) dans la molette, ces pions bloquant ou libérant à leur tour un plongeur (315) bloquant ou relâchant un clapet à billes (221) prévu sur le canal axial (218).

26. Appareil d'injection selon la revendication 25, caractérisé en ce que la molette coulissante coopère avec au moins un pion (332) ou une bille (334) et une lame de ressort (328) dont l'extrémité immobilise le piston de travail (237).

27. Appareil d'injection selon la revendication 17, caractérisé en ce que le piston de travail présente lui-même la forme d'un manchon creux et comporte un prolongement tubulaire directement soumis à l'action du ressort de commande.

**Patentansprüche**

1. Gerät zur nadellosen Injektion von Flüssigkeit mit einem länglichen Körper (1-201-401) aus zwei voneinander trennbaren, aneinanderstoßend angeordneten Teilen, von denen der erste sogenannte medizinische Teil (2-202-402) mindestens einen Sitz (7-234-432) zur Aufnahme einer dichten Kapsel (8-235-431), welche die zu injizierende Flüssigkeit enthält, und mindestens eine Dosierkammer (38-243-430) mit veränderlichem Volumen aufweist, die im ersten Teil ausgebildet ist und mit einer Düse (33-207) in Verbindung steht, die mit einem eigentlichen Injektor zur bildung eines engen Flüssigkeitsstrahls nach einer plötzlichen kräftigen Flüssigkeitszufuhr aus der Dosierkammer versehen ist, und deren zweiter Teil (3-203-403), der sogenannte Antriebsteil, eine in einer Wechselbewegung bewegliche Anordnung mit einem Arbeitskolben (52-233-420) und einer Steuer- und Schubfeder (62-241-244) aufweist, die auf diesen Kolben wirkt und unter der Wirkung der Verschiebung eines Stößels (52-276-445) zunehmend komprimiert wird, die durch einen Druck eines Antriebsfluids in einer im Körper vorgesehenen dichten Kammer (51-287) bewirkt wird, dadurch gekennzeichnet, daß die Kapsel einen beweglichen boden aufweist, dessen Verschiebung die Förderung von Flüssigkeit von dieser Kapsel zur Dosierkammer bewirkt, daß das Antriebsfluid Druckluft ist und daß die kontinuierliche Verschiebung des Stößels bis zu dem Augenblick erfolgt, wo die Feder automatisch und plötzlich mittels eines beweglichen Elements (83-270, 271, 272 - 480, 459, 460) mit begrenzter Verschiebbarkeit freigesetzt wird, was die rasche Rückkehr des Kolbens zugleich mit dem Austreiben von Flüssigkeit aus der Dosierkammer ermöglicht, bevor der Stößel in seine Ausgangsstellung zurückkehrt.

2. Gerät nach Anspruch 1, dadurch gekennzeichnet, daß die bewegliche Anordnung einen Arbeitskolben aufweist, der auch die Funktion des Stößels ausübt, wobei der Kolben (52) den Innenraum des Körpers (1) im zweiten Teil in zwei auf der einen und anderen Seite des Arbeitskolbens angeordnete Kammern (51-119) unterteilt, von denen die erste Kammer teilweise durch den beweglichen boden (13) der Kapsel begrenzt ist und zunehmend unter den Druck von Druckluft gesetzt wird, um den Arbeitskolben in umgekehrter Richtung gegen die Wirkung einer in der zweiten Kammer angeordneten Antriebsfeder (62) zu verschieben, wobei diese Feder die rasche Rückkehr des Arbeitskolbens bewirkt, nachdem der Druck in der ersten Kammer unter der Wirkung eines zum Arbeiskolben koaxialen Verteilers (83) plötzlich verringert wurde, der durch den Kolben am Ende seiner Laufstrecke um eine sehr geringe Strecke verschoben wird, um den Druck in der ersten Kammer an die Umgebungsluft abzulassen, wobei der Arbeitskolben bei seinem raschen Rücklauf seine bewegung auf den Austreibkolben überträgt, der die Flüssigkeit aus der Dosierkammer zum Injektor der Düse drückt.

3. Gerät nach Anspruch 1, dadurch gekennzeichnet, daß der Austreibkolben (40), der die Flüssigkeit aus der Dosierkammer (38) austreibt, einen mit einem Dichtelement (39) versehenen Kopf aufweist, der in der Kammer verschiebbar montiert ist, wobei der Kopf durch eine Stange (44) verlängert ist, die zur Einstellung ihrer Länge gegebenenfalls aus mehreren aufeinanderfolgenden Elementen besteht, und daß der Austreibkolben (40) unter der dauernden Wirkung einer Hilfsfeder (43) steht, die die Stange gegen ein koaxiales Ansatzstück (45) drückt, das mit dem Arbeitskolben (52) fest verbunden ist und mit dem Ende der Stange in berührung steht, wenn die zwei Teile (2-3) des Körpers zusammengesetzt sind.

4. Gerät nach einem der Ansprüche 1-3, dadurch gekennzeichnet, daß der boden des Sitzes (7) des ersten Teils (2) des Körpers (1), der die Kapsel (8) aufnimmt, eine Punktionsnadel (Trocar) (21) aufweist, welche eine die Kapsel abschließende weiche Abdichtungsscheibe (11) durchstoßen kann, um den Austritt der Flüssigkeit zu ermöglichen, welche durch den beweglichen boden (13) der Kapsel herausgedrückt wird, der durch die in die erste Kammer (51) des zweiten Teils (3) des Körpers eingeleitete Druckluft verschoben wird.

5. Gerät nach Anspruch 4, dadurch gekennzeichnet, daß die Punktionsnadel (21) am Ende eines Kanals montiert ist, dessen entgegengesetztes Ende durch ein Kugelventil (24) verschlossen ist, welches den Rücklauf von Flüssigkeit in die Kapsel (8) bei der Förderung der Flüssigkeit aus der Dosierkammer (38) verhindert.

6. Gerät nach einem der Ansprüche 1 bis 5, dadurch gekennzeichnet, daß die Dösierkammer (38) mit dem Injektionskopf (5) durch einen Kanal (28-29) verbunden ist, an dessen Ende ein anderes Kugelventil (31) montiert ist, das unter der Wirkung einer Feder (32) steht und unter dem Druck der aus der Dosierkammer herausgedrückten Flüssigkeit öffnet.

7. Gerät nach Anspruch 6, dadurch gekennzeichnet, daß der Injektionskopf (5) in gerader Richtung und stromabwärts vom Kugelventil (31) einen Halter für eine abnehmbare Düse (33) aufweist, welche einen Injektor (36) aus einem sehr harten und abriebfesten Material trägt, der am Ausgang des Kopfes unter Druck den engen Strahl liefert.

8. Gerät nach einem der Ansprüche 1 bis 5, dadurch gekennzeichnet, daß die Dosierkammer durch einen in einem länglichen Ansatz von sehr geringem Durchmesser (121) vorgesehenen Kanal (122) mit einem flachen Injektor (124) direkt verbunden ist, der möglichst nahe an der äußeren Oberfläche des Injektionskopfes (5) angeordnet ist.

9. Gerät nach Anspruch 1, dadurch gekennzeichnet, daß die zwei Teile (2 und 3) des Körpers (1) durch einen Gewindering (4) verbunden sind, der von einem der Teile (2) getragen und daran arretiert ist und mit einem Schraubgewinde zusammenwirkt, das am Ende eines Verschlußteils (46) ausgebildet ist, um das gegenüberliegende Ende des anderen Teils (3) zu verschließen.

10. Gerät nach einem der Ansprüche 3 bis 9, dadurch gekennzeichnet, daß der Arbeitskolben (52) in seiner dem ersten Teil (2) des Körpers (1) zugewandten Seite eine Innennut (53) zur Aufnahme des Kopfes (54) des Ansatzstücks (45) aufweist, das auf die den Austreibkolben (40) verlängernde Stange (44) wirkt.

11. Gerät nach einem der Ansprüche 1 bis 10, dadurch gekennzeichnet, daß der Verteiler (83) ein abgestuftes zylindrisches Profil (84, 85, 86, 87, 88) aufweist und axial beweglich und mit Ringspielräumen in einem in einem Trägerblock vorgesehenen Durchlaß (82) montiert ist, wobei der Trägerblock mit der Innenwand des zweiten Teils (3) des Körpers (1) durch Arretier- und Sperrelemente (66, 73, 75) fest verbunden ist, wobei der Verteiler mit quer verlaufenden Anschlagflächen von Dichtungsscheiben (77, 78, 79, 80), die im Trägerblock montiert sind, jeweils radiale Spielräume (96, 97) und (96a, 97a) begrenzt, die abwechselnd entsprechend dem axialen Teil des Verteilers das Unter-Druck-Setzen der ersten Kammer (51) oder das Entweichen von Druck aus dieser an die freie Umgebung ermöglichen.

12. Gerät nach Anspruch 11, dadurch gekennzeichnet, daß das Ablassen von Druck in der ersten Kammer (51) an die Umgebungsluft durch einen regelbaren Schlitz (107) erfolgt, dessen Abmessung nach Wunsch zur Regelung des Drucks des zu injizierenden Flüssigkeitsstrahls eingestellt wird mittels eines Ringes (109), der auf eine Verlängerung (72) des Trägerblocks (65) aufschraubbar ist und einen konischen Teil (108) aufweist, der teilweise in einen zwischen der Verlängerung (72) und einem zweiten Ring (75), der auf das Ende des zweiten Teils (3) des Körpers (1) aufgeschraubt ist, begrenzten Hohlraum (106) eindringt.

13. Gerät nach einem der Ansprüche 11 oder 12, dadurch gekennzeichnet, daß die Druckluft in die erste Kammer (51) zwischen dem Arbeitskolben (52) und dem Verschlußstück (46) durch eine axiale Leitung (99) eingeleitet wird, die in einer bohrung (100) des Verteilers (53) verschiebbar und mit dem im Trägerblock (65) vorgesehenen Durchlaß (82) durch einen quer durch den Verteiler ausgebildeten radialen Durchlaß (101) verbunden ist.

14. Gerät nach einem der Ansprüche 11 bis 13, dadurch gekennzeichnet, daß der zwischen dem Verteiler (83) und dem Trägerblock (65) vorgesehenen Durchlaß (82) durch ein Sicherheitsventil (116) mit der Umgebungsluft verbindbar ist, das durch einen außen auf dem Antriebsteil (3) des Körpers (1) montierten verschiebbaren Ring (112) betätigbar ist, wobei der Ring eine innere Ausnehmung (114) aufweist, die in einer Schrägfläche (115) endet, so daß der Ring je nach seiner axialen Stellung das Ventil (116) öffnet oder geschlossen läßt, welches seinerseits durch eine Feder (118) gegen einen Dichtungssitz gedrückt wird.

15. Gerät nach Anspruch 11, dadurch gekennzeichnet, daß der Verteiler (83) unter Wirkung einer Rückstellfeder (98) steht, welche den Verteiler gegen die Dichtungsscheiben (77 und 79) andrückt, welche gegen das Entweichen von Druckluft in die erste Kammer (51) abschließen, solange der Arbeitskolben (52) nicht genügend verschoben ist, um den Verteiler gegen die Rückstellfeder zurückzudrücken.

16. Gerät nach einem der Ansprüche 1 bis 15, dadurch gekennzeichnet, daß es mit einer Druckluftquelle verbunden ist, die durch ein betätigungselement gesteuert ist, so daß sie diese Druckluft in die erste Kammer (51) bis zur Verschiebung des beweglichen Verteilers (83) abgibt, die am Ende der Laufstrecke durch den Arbeitskolben (52) gesteuert ist und die Unterbrechung der Druckluftzufuhr in die Kammer bewirkt, wobei eine erneute Drucklufteinleitung zur Durchführung des folgenden Zyklus eine Wiederholung des Steuerungsbefehls des Elements erfordert.

17. Gerät zur nadellosen Injektion von Flüssigkeit nach Anspruch 1, dadurch gekennzeichnet, daß die im zweiten Teil (203) des Körpers (201) montierte bewegliche Anordnung einen Arbeitskolben (237) aufweist, welcher unter der Wirkung einer Steuerfeder (241) steht, die durch einen axialen Druck ausübenden Stößel (276) zunehmend zusammengedrückt wird, wobei der Stößel durch eine elastische Membran (280) mit der Innenwand einer mit einer Druckluftquelle verbundenen Druckkammer (287) verbunden ist, und eine Vorrichtung aufweist, um den Arbeitskolben fest mit einem im Körper koaxial zum Kolben vorgesehenen Element zur vorübergehenden Feststellung (269-270) zu verbinden, so daß dieses, jenseits einer bestimmten Laufstrecke des Stößels, welche das zunehmende Zusammendrücken der Feder bewirkt, plötzlich freigesetzt wird und die rasche Verschiebung des Kolbens veranlaßt, wobei aus einer Dosierkammer (243), die mit einer den Flüssigkeitsvorrat bildenden Kapsel (235) in Verbindung steht, zu injizierende Flüssigkeit ausgetrieben wird.

18. Injektionsgerät nach Anspruch 17, dadurch gekennzeichnet, daß die blockiervorrichtung aus Kugeln (270) besteht, die in in der Außenwand des Kolbens (237) vorgesehenen offenen Sitzen (269) angeordnet und durch eine koaxiale Hülse (272) in ihrer Stellung gehalten sind, die auf dieser Wand unter der Wirkung des Stößels verschiebbar ist und eine Profilausnehmung (273) aufweist, um das Entweichen der Kugeln und die Freigabe des Kolbens jenseits einer gegebenen Laufstrecke der Hülse zu ermöglichen.

19. Injektionsgerät nach einem der Ansprüche 17 oder 18, dadurch gekennzeichnet, daß der Arbeitskolben (237) eine koaxiale Hülse (233) aufweist und bezüglich eines feststehenden axialen Dorns (217) verschiebbar montiert ist und eine Ringkammer (243) zur Dosierung von Flüssigkeit zwischen dem Dorn und der Hülse begrenzt, wobei diese Kammer mit einem axialen Kanal (218) in Verbindung steht, der im Dorn in der Verlängerung eines Schnabels (205) vorgesehen ist, der am Ende mit einem Flüssigkeitsinjektor (207) versehen ist, der unter der Wirkung des durch die Verschiebung des Arbeistkolbens (237) erzeugten Drucks einen schmalen Strahl bildet.

20. Injektionsgerät nach Anspruch 19, dadurch gekennzeichnet, daß der axiale Kanal ein Kugelventil (231) aufweist, das gegenüber einer mit dem Innenraum der Kapsel (235) in Verbindung stehenden Hohlnadel (229) angeordnet ist, um den Zutritt von Flüssigkeit in die Dosierkammer (243) durch im Dorn (217) ausgebildete radiale Kanäle (244) zu ermöglichen, wobei das Ventil die Verbindung mit der Kapsel bei Verschiebung des Arbeitskolbens (237) unter der Wirkung der Steuerfeder (241) verschließt.

21. Injektionsgerät nach einem der Ansprüche 17 bis 20, dadurch gekennzeichnet, daß die zum Arbeitskolben koaxiale Hülse (233) mit einem handbetätigten Sperrhebel (247) zusammenwirkt, der um eine Achse (246) am Körper (201) schwenkbar ist und ein Endstück (248) aufweist, das in einen offenen Sitz (249) der Hülse (233) eingreifen kann, um deren Verschiebung bezüglich des feststehenden Dorns (217) zu verhindern.

22. Injektionsgerät nach einem der Ansprüche 17 bis 21, dadurch gekennzeichnet, daß die Kapsel einen beweglichen boden (254) aufweist, der unter der Wirkung eines Druckkolbens (255) steht, welcher dauernd der Wirkung von Federn (260) unterworfen ist, die rings um eine hohle äußere Hülse (259) montiert sind, in welcher der Arbeitskolben verschiebbar ist, wobei diese Federn am Ende mit einem Querstück (257) verbunden sind, das in einem Schlitz (266) der Hülse verschiebbar ist.

23. Injektionsgerät nach Anspruch 18, dadurch gekennzeichnet, daß der Arbeitskolben (237) eine in der Hülse (272) axial bewegliche rohrförmige Verlängerung (268) aufweist.

24. Injektionsgerät nach Anspruch 19, dadurch gekennzeichnet, daß der Körper (201) einen Schlitz (303) für die Verschiebung einer Querplatte (300) aufweist, die durch einen Druckknopf (298) betätigbar ist und eine Mittelöffnung (302) aufweist, welche die Kontinuität des Kanals (218) für das Austreten von von der Dosierkammer kommender Flüssigkeit zum Injektor nur dann ermöglicht, wenn der Druckknopf in den Schlitz des Körpers eingedrückt ist.

25. Injektionsgerät nach Anspruch 17, dadurch gekennzeichnet, daß das Element zur vorübergehenden Feststellung des Arbeitskolbens einen auf der Außenfläche des Körpers (201) verschiebbaren Steuerschieber aufweist, um die Köpfe (313) von radialen Stiften (311) bezüglich einer Ausnehmung (324) im Schieber festzustellen oder freizugeben, wobei diese Stifte ihrerseits einen Tauchstift (315) blockieren oder freilassen, der ein im axialen Kanal (218) vorgesehenes Kugelventil blockiert oder freigibt.

26. Injektionsgerät nach Anspruch 25, dadurch gekennzeichnet, daß der verschiebbare Steuerschieber mit mindestens einem Stift (332) oder einer Kugel (334) und einer blattfeder (328) zusammenwirkt, deren Ende den Arbeitskolben (237) feststellt.

27. Injektionsgerät nach Anspruch 17, dadurch gekennzeichnet, daß der Arbeitskolben selbst die Form einer hohlen Hülse besitzt und eine rohrförmige Verlängerung aufweist, welche direkt von der Steuerungsfeder beaufschlagt ist.

## Claims

1. A needle-less device for injecting of liquid, comprising an elongate body (1 - 201 - 401) in two separable parts placed end to end, the first of which, called the medical part (2 - 202 - 402), comprises at least one housing (7 - 234 - 432) which receives a sealed cartridge (8 - 235 - 431) containing the liquid to be injected and at least one variable volume dosing chamber (38 - 243 - 430), formed in the said first part and communicating with a nozzle (33 - 207) equipped with an injector suitable for forming a narrow jet of liquid as a result of a sudden expulsion of liquid from the dosing chamber, and the second part of which, called the driving part (3 - 203 - 403), comprises a movable assembly having an alternating movement which comprises an operating piston (52 - 233 - 420) and a control and propulsion spring (62 - 241 - 444) which acts on the piston when being progressively compressed as a result of the displacement of a pushing element (52 - 276 - 445) caused by the pressure of a propulsion fluid in a sealed chamber (51 - 287) provided in the body, characterized in that the cartridge has a movable base the displacement of which causes the movement of liquid from this cartridge to the dosing chamber, in that the propulsion fluid is compressed air and in that the continuous displacement of the pushing element occurs until the time when the spring is automatically and suddenly freed by means of a movable element (83 - 270, 271, 272 - 458, 459, 460) having a limited displacement which allows the rapid return of the piston simultaneously with the expulsion of liquid from the dosing chamber prior to the return of the pushing element to its initial position.

2. A device according to claim 1, characterized in that the movable assembly comprises an operating piston which also acts as the pushing element, the piston (52) separating the interior of the said second part of the body (1) into two chambers (51 - 119) disposed on either side of the said operating piston, the first chamber being partially delimited by the movable base (13) of the cartridge and being progressively subjected to compressed air pressure so as to displace the operating piston in the opposite direction against the action of a propulsion spring (62) housed in the second chamber, the said spring causing the rapid return of the operating piston as a result of a sudden reduction in pressure in the said first chamber controlled by a distributor (83) coaxial with the operating piston and displaced over a very short distance by the said piston at the end of its travel in such a manner as to allow the pressure in the said first chamber to escape to the open air, the operating piston during its rapid return transmitting its movement to the expulsion piston which forces the liquid out of the dosing chamber towards the injector in the nozzle.

3. A device according to claim 1, characterized in that the piston (40) for expelling liquid from the dosing chamber (38) comprises a head, equipped with a sealing element (39) and slidingly mounted in the said chamber, the said head being prolonged by a shaft (44), optionally made of several successive elements in order to adjust the length of the said shaft, the said expulsion piston (40) being subjected to the constant action of an auxiliary spring (43) which pushes the shaft back against a coaxial connecting piece (45) which is fixed to the operating piston (52) and is in contact with the end of the shaft when the two parts (2 - 3) of the body are assembled.

4. A device according to any one of claims 1 to 3, characterized in that the base of the housing (7) of the first part (2) of the body (1) receiving the cartridge (8) comprises a trocar (21), adapted to pass through a supple sealing washer (11) closing the said cartridge, to allow the exit of liquid expelled by the movable base (13) of the cartridge, displaced by the compressed air admitted into the first chamber (51) of the second part (3) of the body.

5. A device according to claim 4, characterized in that the trocar (21) is mounted at the end of a channel the opposite end of which is closed by a ball valve (24) which prevents the return of liquid into the cartridge (8) during the expulsion of liquid from the dosing chamber (38).

6. A device according to any one of claims 1 to 5, characterized in that the dosing chamber (38) is connected to the injection head (5) by a channel (28 - 29) at the end of which another ball valve (31), subject to the action of a spring (32), is mounted, the opening of the said valve being caused by the pressure of liquid forced out of the dosing chamber.

7. A device according to claim 6, characterized in that the injection head (5) comprises, in line with and in front of the ball valve (31), a support for a detachable nozzle (33) car tying an injector (36), made of very hard and abrasion resistant material, supplying the narrow jet under pressure at the outlet from the head.

8. A device according to any one of claims 1 to 5, characterized in that the dosing chamber is directly connected by a channel (122), provided in an elongate connecting element having a very small diameter (121), to a flat injector (124) disposed as close as possible to the external surface of the injection head (5).

9. A device according to claim 1, characterized in that the two parts (2 and 3) of the body (1) are connected by a threaded ring (4) located and held fixed on one of the two parts (2) and cooperating with a thread formed on the end of a closing part (46) which closes off the facing end of the other part (3).

10. A device according to any one of claims 3 to 9, characterized in that the operating piston (52) comprises in its face directed towards the first part (2) of the body (1) an internal groove (53) receiving the head (54) of the connecting element (45) which acts on the shaft (44) prolonging the expulsion piston (40).

11. A device according to any one of claims 1 to 10, characterized in that the distributor (83) has a stepped cylindrical profile (84, 85, 86, 87, 88) and is mounted to be axially movable and with annular clearances in a passage (82) provided in a support block (65), fixed to the internal wall of the second part (3) of the body (1), by holding and locking means (66, 73, 75), the said distributor delimiting, together with the transverse bearing surfaces of butt joints (77, 78, 79, 80) mounted in the support block, radial clearances (96, 97) and (96a, 97a) respectively, alternatively allowing, depending on the axial position of the distributor, the pressurizing of the first chamber (51) or the escape of this pressure to the open air.

12. A device according to claim 11, characterized in that the escape to the open air of the pressure in the first chamber (51) occurs through an adjustable slot (107) the size of which is adjusted as required, in order to act on the pressure of the jet of liquid to be injected, by means of a ring (109) adapted to be threaded onto an extension (72) of the support block (65), the said ring comprising one part (108) having a conical shape which partially enters into a cavity (106) delimited between the said extension (72) and a second ring (75) threaded onto the end of the second part (3) of the body (1).

13. A device according to one of claims 11 or 12, characterized in that the compressed air is admitted into the first chamber (51) between the operating piston (52) and the closing part (46) through a coaxial duct (99) sliding in a bore (100) in the distributor (83) and connected to the passage (82) provided in the support block (65) by means of a radial channel (101) formed across the distributor,

14. A device according to any one of claims 11 to 13, characterized in that the passage (82) provided between the distributor (83) and the support block (65) can be connected to the open air by means of a safety valve (116) operated by a slidable ring (112) mounted on the outside of the driving part (3) of the body (1), the said ring comprising an internal recess (114) terminated by a sloping face (115) such that, depending on its axial position, it opens or leaves closed the said valve (116) which is itself applied against a sealing seat by a spring (118).

15. A device according to claim 11, characterized in that the distributor (83) is subject to the action of a return spring (98) which applies the said distributor against the butt joints (77 and 79) which close the escape route for the pressurized air in the first chamber (51) so long as the operating piston (52) is not sufficiently displaced to force back the said distributor against the action of the said return spring.

16. A device according to any one of claims 1 to 15, characterized in that it is connected to a source for supplying compressed air controlled by an operating element which delivers this air into the first chamber (51) until the movable distributor (83) is displaced at the end of its travel by the operating piston (52) and which stops the admission of air into the said chamber, a renewed admission of air in order to perform the following cycle requiring the said element to be operated again.

17. A device for injection of liquid according to claim 1, characterized in that the movable assembly mounted in the second part (203) of the body (201) comprises an operating piston (237) subject to the action of a control spring (241) progressively compressed by an axial pushing element (276) connected by an elastic membrane (280) to the internal wall of a compression chamber (287) connected to a source of compressed air, and means for fixing the operating piston to an element for temporary immobilization (269 - 270) provided in the body, coaxial with the piston, in such a manner that, beyond a predetermined travel of the pushing element causing the progressive compression of the spring, the latter is suddenly released and causes the rapid displacement of the piston whilst causing the expulsion from a dosing chamber (243) of the liquid to be injected which communicates with a cartridge (235) forming a liquid storage element.

18. An injection device according to claim 17, characterized in that the means for locking are constituted by balls (270) mounted in recesses (269) provided in the external wall of the piston (237) and held in position by a coaxial sleeve (272) sliding along this wall as a result of the action of the pushing element, this sleeve comprising an opening (273) shaped to allow the balls to escape and to free the piston after a predetermined travel of the sleeve.

19. An injection device according to one of claims 17 or 18, characterized in that the operating piston (237) comprises a coaxial sleeve (233) and is mounted to slide relative to a fixed hollow shaft (217) delimiting an annular liquid dosing chamber (243) between the hollow shaft and the sleeve, this chamber communicating with an axial channel (218) provided in the hollow shaft in the prolongation of a nose (205) equipped at the end with a liquid injector (207) which forms a narrow jet as a result of the pressure created by the displacement of the operating piston (237).

20. An injection device according to claim 19, characterized in that the axial channel comprises a ball valve (231) provided facing a trocar (229) which communicates with the interior of the cartridge (235) to allow the admission of liquid into the dosing chamber (234) through radial ducts (244) made in the hollow shaft (217), the said valve blocking the communication with the cartridge when the operating piston (237) is displaced under the action of the control spring (241).

21. An injection device according to any one of claims 17 to 20, characterized in that the sleeve (233) which is coaxial with the operating piston cooperates with a manually controlled locking lever (247) pivotable around a shaft (246) on the body (201), this lever comprising a claw (248) adapted to engage in a recessed seat (249) in the sleeve (233) to prevent it sliding relative to the fixed hollow shaft (217).

22. An injection device according to any one of claims 17 to 21, characterized in that the cartridge comprises a movable base (254) which can be moved as a result of the action of a pushing piston (255) constantly subjected to the action of springs (260) mounted around a hollow external sleeve (259) in which the operating piston slides, these springs being fixed to the end of a crosspiece (257) which moves in a slot (266) in the sleeve.

23. An injection device according to claim 18, characterized in that the operating piston (237) comprises a tubular prolongation (268) which is axially movable in the sleeve (272).

24. An injection device according to claim 19, characterized in that the body (201) comprises a slot (303) for the displacement of a transverse plate (300) operated by a push-button (298), this plate comprising a central passage (302) making it possible to ensure the continuity of the channel (218) for the escape of liquid coming from the dosing chamber towards the injector only when the push-button is pushed towards the interior of the slot in the body.

25. An injection device according to claim 17, characterized in that the element for temporary immobilisation of the operating piston comprises a regulator sliding on the external surface of the body (201) in order to immobilise or free the heads (313) of radial pegs (311) relative to a recess (324) in the regulator, these pegs in their turn blocking or freeing a plunger (315) which blocks or frees a ball valve (221) provided on the axial channel (218).

26. An injection device according to claim 25, characterized in that the sliding regulator cooperates with at least one peg (332) or one ball (334) and a spring blade (328) the end of which immobilizes the operating piston (237).

27. An injection device according to claim 17, characterized in that the operating piston itself has the shape of a hollow sleeve and comprises a tubular prolongation which is directly subjected to the action of the control spring.

FIG. 1

FIG. 4

FIG. 3

EP 0 294 272 B1

## FIG. 2 a

## FIG. 2 b

EP 0 294 272 B1

FIG. 5

EP 0 294 272 B1

FIG. 6a

FIG. 6b

EP 0 294 272 B1

FIG. 7

FIG. 8

FIG. 9

FIG. 10

FIG. 11

FIG. 12

EP 0 294 272 B1

FIG. 13

FIG. 16

FIG. 14

FIG. 15

FIG. 17

EP 0 294 272 B1

FIG. 18 a

FIG. 18 b

29